# EUROPEAN PATENT APPLICATION

(11) **EP 4 365 164 A1**
(43) Date of publication of application: **08.05.2024**
(21) Application number: 22833647.5
(22) Date of filing: 29.06.2022
(51) Int. Cl.: C07C 233/57, C07D 211/32, C07D 401/10, C07D 309/08, C07D 407/12, C07D 335/02, C07D 409/12, C07D 295/14, C07D 209/10, C07D 403/10

(54) **NOVEL COMPOUND FOR CAPPING LAYER, AND ORGANIC LIGHT-EMITTING DEVICE COMPRISING SAME**

(30) Priority: 30.06.2021 KR 20210086131; 26.01.2022 KR 20220011861
(71) Applicant: Dongjin Semichem Co., Ltd, Incheon 22824 (KR); Samsung Display Co., Ltd., Yongin-si, Gyeonggi-do 17113 (KR)
(72) Inventor: HAM, Ho Wan, Hwaseong-si Gyeonggi-do 18635 (KR); AN, Hyun Cheol, Hwaseong-si Gyeonggi-do 18635 (KR); KIM, Hee Joo, Hwaseong-si Gyeonggi-do 18635 (KR); KIM, Dong Jun, Hwaseong-si Gyeonggi-do 18635 (KR); HAN, Jeong Woo, Hwaseong-si Gyeonggi-do 18635 (KR); LEE, Hyung Jin, Hwaseong-si Gyeonggi-do 18635 (KR); ANN, Ja Eun, Hwaseong-si Gyeonggi-do 18635 (KR); KWON, Dong Yuel, Hwaseong-si Gyeonggi-do 18635 (KR); CHO, Ill Hun, Yongin-si, Gyeonggi-do 17113 (KR); LEE, Bo Ra, Yongin-si, Gyeonggi-do 17113 (KR); PARK, Yeong Rong, Yongin-si, Gyeonggi-do 17113 (KR); OH, Il Soo, Yongin-si, Gyeonggi-do 17113 (KR); LEE, Dae Woong, Yongin-si, Gyeonggi-do 17113 (KR); IM, Hyeon Jeong, Yongin-si, Gyeonggi-do 17113 (KR)
(74) Representative: Grünecker Patent- und Rechtsanwälte PartG mbB
(86) International application number: PCT/KR2022/009366
(87) International publication number: WO 2023/277593

(57) **Abstract**

A novel compound for a capping layer, and an organic light-emitting device containing the same are proposed.

## Description

### BACKGROUND OF THE DISCLOSURE

### 1. Field of the Disclosure

The present disclosure relates to a novel compound for a capping layer and an organic light emitting device including the same.

### 2. Description of the Related Art

Materials used for organic layers in organic light emitting devices can be classified into light emitting materials, hole injection materials, hole transport materials, electron transport materials, electron injection materials, and the like, according to the functions thereof.

The light emitting materials can be classified into a fluorescent material derived from a singlet excited state of an electron and a phosphorescent material derived from a triplet excited state of an electron according to the light emitting mechanisms and also classified into blue, green, and red light emitting materials according to the emission colors.

A typical organic light emitting device may have a structure in which an anode is disposed on a substrate, and a hole transport layer, a light emitting layer, an electron transport layer, and a cathode are sequentially stacked on the anode. Here, the hole transport layer, the light emitting layer, and the electron transport layer are organic thin films made of organic compounds.

The principle for driving such an organic light emitting device having the structure will be described below.

When a voltage is applied between the anode and the cathode, holes injected from the anode move to the light emitting layer through the hole transport layer, and electrons injected from the cathode move to the light emitting layer through the electron transport layer. The holes and electrons recombine in the light emitting layer to generate excitons.

The holes and electrons recombine in the light emitting layer to generate excitons.

Light is generated when the excitons change from an excited state to a ground state. Regarding the efficiency of the organic light emitting device, internal luminous efficiency and external luminous efficiency are considered. The internal luminous efficiency is related to how efficiently excitons are generated and photoconverted in the organic layers interposed between the anode and cathode, such as the hole transport layer, the light emitting layer, and the electron transport layer. The internal luminous efficiency is theoretically known to be 25% for the fluorescence and 100% for the phosphorescence.

On the other hand, the external luminous efficiency refers to how to efficiently extract the light generated in the organic layers so that the light can exit the organic light emitting device, and it is known that the level of the external luminous efficiency is about 20% of level of the internal luminous efficiency. To increase the light extraction, various organic compounds with a refractive index of 1.7 or more have been used for a capping layer to prevent light from being lost by total reflection. In addition, organic light-emitting devices including a composite capping structure composed of a high refractive index capping layer and a low refractive index capping layer are being developed to further increase the external luminous efficiency. LiF has been commercially used as a capping layer material with low refractive index. However, since inorganic compounds such as LiF have high deposition temperatures and poor processability, efforts to use organic compounds rather than inorganic compounds are being made. Boron coordination compounds are known as materials with a low refractive index, but the boron coordination compounds suffer low stability which causes problems such as reducing the lifetime of organic light-emitting devices. Therefore, efforts are being continuously made to develop organic capping layer materials that maintain a low refractive index and have good compound stability.

### SUMMARY OF THE DISCLOSURE

The present disclosure is to provide a compound for a capping layer and an organic light-emitting device including the same, in which the compound has a structure in which two or more amine or carbonyl substituents have a structure connected through a cycloalkylene group, so that a low refractive index can be formed and, in particular, a broad band gap can be maintained, thereby enabling a lower extinction coefficient even in a short wavelength range and providing a low refractive index.

Additionally, the present disclosure is to provide a compound for a capping layer and an organic light-emitting device including the same, in which the compound has a substituent having a low polarizability, which have a lower refractive index, thereby being highly effective in improving the efficiency and color purity of an organic light-emitting device.

Additionally, the present disclosure is to provide a compound for a capping layer and an organic light-emitting device including the same, in which the compound has thermal stability due to a structure including a cycloalkylene linker and has excellent film orientation due to a structure in which two or more amine-based or carbonyl-based substituents are bonded through a cycloalkylene linker, and thus has improved stability against contamination from external oxygen, air, moisture, etc. Therefore, when the compound is used to form a capping layer of an organic light-emitting device, the capping layer highly effectively improve the lifespan of the organic light-emitting device.

The above-mentioned objectives and other objectives will be understood from the description provided below.

To address the above challenges, in one aspect, the present disclosure provides a compound for a capping layer, the compound being represented by the following Formula 1: 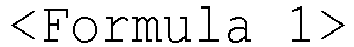 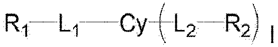 In Formula 1,
Cy is a substituted or unsubstituted C3~C50 cycloalkylene group, or a C3~C50 cycloalkenylene group,
L and L1 are each independently a direct bond, a substituted or unsubstituted C1~C50 alkylene group, a substituted or unsubstituted C2~C50 alkenylene group, a substituted or unsubstituted C1~C50 alkyleneoxy group, an ether group, a substituted or unsubstituted C1~C50 sulfide group, a thioether group, a substituted or unsubstituted C1~C50 carbonyl group, substituted or unsubstituted -C(X1)NR3-, substituted or unsubstituted -NR4C(X2)-, substituted or unsubstituted -NR-, a substituted or unsubstituted C3~C50 cycloalkylene group, a substituted or unsubstituted C1~C50 heterocyclylene group, or a combination thereof,
X1 and X2 are each independently O, S, Se, Te, NR5, or CR6R7,
R and R1 to R7 are each independently hydrogen, deuterium, halogen, a nitro group, a nitrile group, a hydroxy group, a thiol group, a substituted or unsubstituted amino group, a substituted or unsubstituted C1 to C50 alkyl group, a substituted or unsubstituted C2~C50 alkenyl group, a substituted or unsubstituted C1~C50 alkoxy group, a substituted or unsubstituted C1~C50 sulfide group, a substituted or unsubstituted silyl group, a substituted or unsubstituted C3~C50 cycloalkyl group, a substituted or unsubstituted C1~C50 heterocyclyl group, a substituted or unsubstituted C3~C50 aryl group, or a substituted or unsubstituted C2~C50 heteroaryl group, and a plurality of adjacent R, R1 to R7 form or not form a ring by bonding with each other,
l is an integer in a range of from 1 to 5.

In addition, in one embodiment, there is provided an organic light-emitting device including the compound described above.

The compound for a capping layer according to one embodiment of the present disclosure has a structure in which two or more amine or carbonyl substituents have a structure connected through a cycloalkylene group, so that a low refractive index can be formed and, in particular, a broad band gap can be maintained, thereby enabling a lower extinction coefficient even in a short wavelength range and providing a low refractive index.

In addition, the compound has a lower refractive index because the compound includes a substituent having a low polarizability, thereby being capable of highly effectively improving the efficiency and color purity of organic light-emitting devices.

In addition, the compound for a capping layer has thermal stability due to a structure including a cycloalkylene linker and has excellent film orientation due to a structure in which two or more amine-based or carbonyl-based substituents are bonded through a cycloalkylene linker, and thus has improved stability against contamination from external oxygen, air, moisture, etc. Therefore, when the compound is used to form a capping layer of an organic light-emitting device, the capping layer highly effectively improve the lifespan of the organic light-emitting device.

The above effects and other effects will be described in detail below.

### BRIEF DESCRIPTION OF THE DRAWINGS

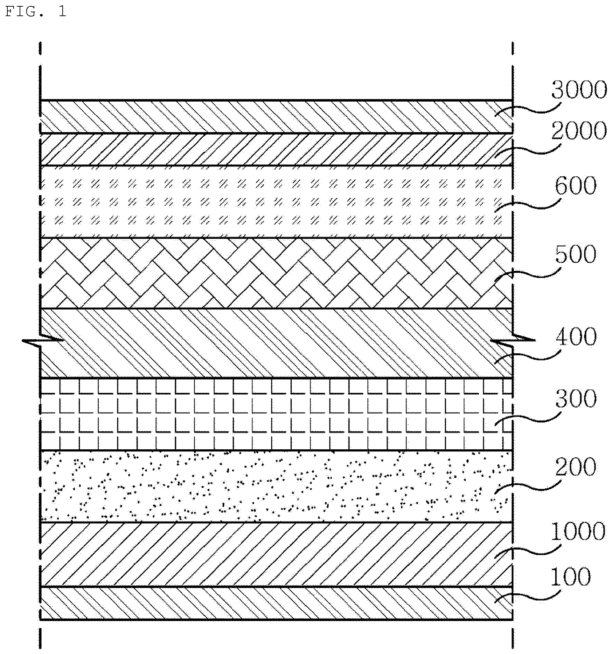

FIG. 1 is a schematic cross-sectional view illustrating the construction of an organic light-emitting device according to one embodiment of the present disclosure.

### ** Explanation of drawing symbols **

100: substrate
200: Hole injection layer
300: Hole transport layer
400: light emitting layer
500: electron transport layer
600: Electron injection layer
1000: first electrode
2000: Second electrode
3000: capping layer

### DESCRIPTION OF THE PREFERRED EMBODIMENTS

Prior to a description of the present disclosure, it should be noted that the terms used in the present specification are used only to describe specific examples and are not intended to limit the scope of the present disclosure which will be defined only by the appended claims.

Unless otherwise defined herein, all terms including technical and scientific terms used herein have the same meaning as commonly understood by those who are ordinarily skilled in the art to which the present disclosure pertains.

Unless otherwise stated herein, it will be further understood that the terms "comprise", "comprises", and "comprising", when used in this specification, specify the presence of stated features, regions, integers, steps, operations, elements and/or components but do not preclude the presence or addition of one or more other features, regions, integers, steps, operations, elements, components and/or groups thereof.

Throughout the specification and claims of the disclosure, the term "aryl" refers to a functional group having a C5-50 aromatic hydrocarbon ring, and examples thereof include phenyl, benzyl, naphthyl, biphenyl, terphenyl, fluorene, phenanthrenyl, triphenylenyl, perylenyl, chrysenyl, fluoranthenyl, benzofluorenyl, benzotriphenylenyl, benzochrysenyl, anthracenyl, stilbenyl, or pyrenyl. The term "heteroaryl" refers to a C2-50 aromatic ring structure containing at least one heteroatom, and it includes a heterocyclic ring formed from pyrrolyl, pyrazinyl, pyridinyl, indolyl, isoindolyl, furyl, benzofuranyl, isobenzofuranyl, dibenzofuranyl, benzothiophenyl, dibenzothiophenyl, quinolyl group, isoquinolyl, quinoxalyl, carbazolyl, phenanthridinyl, acridinyl, phenanthrolinyl, thienyl, a pyridine ring, a pyrazine ring, a pyrimidine ring, a pyridazine ring, a triazine ring, an indole ring, a quinoline ring, an acridine ring, a pyrrolidine ring, a dioxane ring, a piperidine ring, a morpholine ring, a piperazine ring, a carbazole ring, a furan ring, a thiophene ring, an oxazole ring, an oxadiazole ring, a benzofuran ring, a thiazole ring, a thiadiazole ring, a benzothiophene ring, a triazole ring, an imidazole ring, a benzoimidazole ring, a pyran ring, or a dibenzofuran ring.

In chemical formulas: Arx (where x is an integer) means a substituted or unsubstituted C6-C50 aryl group, or a substituted or unsubstituted C2-C50 heteroaryl group, unless otherwise defined; Lx (where x is an integer) means a directly bonded and substituted or unsubstituted C6-C50 arylene group or a substituted or unsubstituted C2-C50 heteroarylene group, unless otherwise defined; and Rx (where x is an integer), means a hydrogen, deuterium, halogen, a nitro group, a nitrile group, a substituted or unsubstituted C1-C30 alkyl group, a substituted or unsubstituted C2-C30 alkenyl group, a substituted or unsubstituted C1-C30 alkoxy group, a substituted or unsubstituted C1-C30 sulfide group, a substituted or unsubstituted C6-C50 aryl group, or a substituted or unsubstituted C2-C50 heteroaryl group, unless otherwise defined.

Throughout the present specification and claims, the term "substituted or unsubstituted" means that a portion is substituted or unsubstituted by at least one selected from the group consisting of deuterium, halogen, amino groups, cyano groups, nitrile groups, nitro groups, nitroso groups, sulfamoyl groups, isothiocyanate groups, thiocyanate groups, carboxyl groups, C1-C30 alkyl groups, C1-C30 alkylsulfinyl groups, C1-C30 alkylsulfonyl groups, C1-C30 alkylsulfanyl groups, C1-C12 fluoroalkyl groups, C2-C30 alkenyl groups, C1-C30 alkoxy groups, C1-C12 N-alkylamino groups, C2-C20 N,N-dialkylamino groups, substituted or unsubstituted C1-C30 sulfide groups, C1-C6 N-alkylsulfamoyl groups, C2-C12 N,N-dialkylsulfamoyl groups, C0-C30 silyl groups, C3-C20 cycloalkyl groups, C3-C20 heterocycloalkyl groups, C6-C50 aryl groups, C3-C50 heteroaryl groups, etc.

In addition, the same symbols throughout the present specification may have the same meaning unless otherwise specified.

All or some embodiments described herein may be selectively combined and configured so that the embodiments may be modified in various ways unless the context clearly indicates otherwise.

Hereinafter, embodiments of the present disclosure and the effects thereof will be described in detail below.

An organic light-emitting device according to an embodiment of the present disclosure may be an organic light-emitting device including a capping layer.

Specifically, the organic light-emitting device may include a first electrode, a second electrode, one or more organic layers disposed between the first electrode and the second electrode, and a capping layer disposed on an outer surface of either the first electrode or the second electrode and made of the compound of the present disclosure.

Specific examples of the compound of the present disclosure include compounds represented Formula 1 shown below. 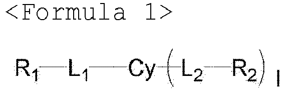 In Formula 1,
Cy is a substituted or unsubstituted C3~C50 cycloalkylene group, or a C3~C50 cycloalkenylene group,
L and L1 are each independently a direct bond, a substituted or unsubstituted C1~C50 alkylene group, a substituted or unsubstituted C2~C50 alkenylene group, a substituted or unsubstituted C1~C50 alkyleneoxy group, an ether group, a substituted or unsubstituted C1~C50 sulfide group, a thioether group, a substituted or unsubstituted C1~C50 carbonyl group, substituted or unsubstituted -C(X1)NR3-, substituted or unsubstituted -NR4C(X2)-, substituted or unsubstituted -NR-, a substituted or unsubstituted C3~C50 cycloalkylene group, a substituted or unsubstituted C1~C50 heterocyclylene group, or a combination thereof, and specifically, it may be a combination of three or less of these,
X1 and X2 are each independently O, S, Se, Te, NR5, or CR6R7,
R and R1 to R7 are each independently hydrogen, deuterium, halogen, a nitro group, a nitrile group, a hydroxy group, a thiol group, a substituted or unsubstituted amino group, a substituted or unsubstituted C1 to C50 alkyl group, a substituted or unsubstituted C2~C50 alkenyl group, a substituted or unsubstituted C1~C50 alkoxy group, a substituted or unsubstituted C1~C50 sulfide group, a substituted or unsubstituted silyl group, a substituted or unsubstituted C3~C50 cycloalkyl group, a substituted or unsubstituted C1~C50 heterocyclyl group, a substituted or unsubstituted C3~C50 aryl group, or a substituted or unsubstituted C2~C50 heteroaryl group, and a plurality of adjacent R, R1 to R7 may or may not form a ring by bonding with each other,
l is an integer in a range of from 1 to 5.

In the above, Cy may specifically be a substituted or unsubstituted C3~C10 cycloalkylene group.

In addition, in the specific examples of the capping layer compound of the present disclosure, the compound of Formula 1 refers a capping layer compound represented by Formula 2 or 3 shown below. 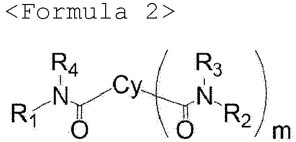 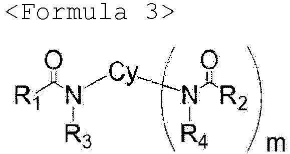 In Formulas 2 to 3, the same symbols as in Formula 1 above have the same definitions as in Formula 1,
m is an integer in a range of from 1 to 5 and can be 1 or 2.

The capping layer compound of the present disclosure represented by Formula 2 or Formula 3 above, maintains a low refractive index through an amide linker and has excellent chemical and thermal stability.

In Formula 1, one or more of R1 and R2 may be a substituted or unsubstituted C3~C50 cycloalkyl group, or a substituted or unsubstituted C1~C50 heterocyclyl group. This structure further improves thermal stability and can simultaneously lower the polarizability to form a low refractive index.

Specifically, R1 and R2 may both be a substituted or unsubstituted C3~C50 cycloalkyl group, or a substituted or unsubstituted C1~C50 heterocyclyl group. This structure may form a lower refractive index by lowering the intramolecular polarizability and at the same time having a low extinction coefficient even in the short wavelength range.

Additionally, at least one of R1 and R2 may be a substituted C3~C50 cycloalkyl group, or a substituted C1~C50 heterocyclyl group. This substituted structure may improve thermal stability.

Substituents of R1 and R2 are each independently selected from the group consisting of hydroxyl groups, thiol groups, amino groups, C1-C30 alkyl groups, C1-C30 alkoxy groups, C1-C30 sulfide groups, silyl groups, halogen groups, C3-C30 cycloalkyl groups, C1-C30 heterocyclyl groups, and combinations thereof, but are not limited thereto. With the inclusion of these substituents, it is possible to maintain a low refractive index and high thermal stability.

Furthermore, in Formula 1, Cy may be selected from Structural Formula Formulas A-1 to A-15 shown below.

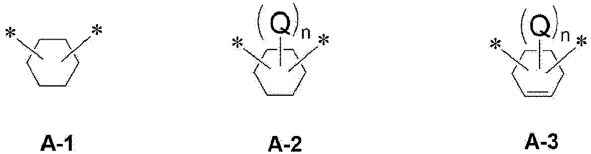

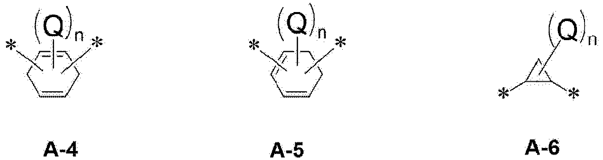

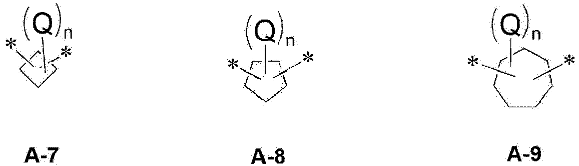

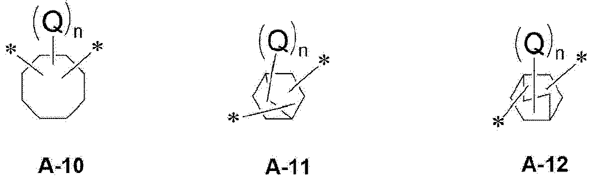

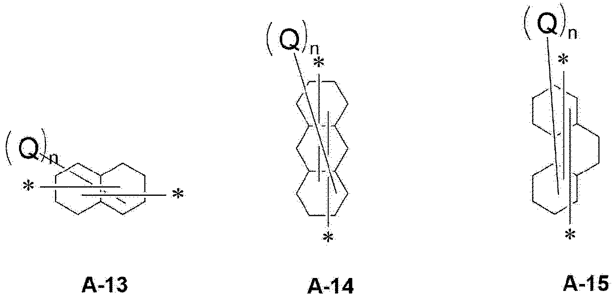

In Structural Formulas A-1 to A-15, Q is each independently a methyl group, an ethyl group, a t-butyl group, a cyclohexyl group, an adamantane group, a dihydroamine group, a dimethylamine group, a hydroxy group, a methoxy group, a mercaptan group, methylthio group, a fluoro group, a trifluoromethyl group, or a trimethylsilyl group,
n is each independently an integer in a range of from 0 to 8, specifically an integer in a range of from 0 to 4,
* indicates a bonding site.

Additionally, in Formula 1, when l is 1, Cy may be a cycloalkylene where -L1-R1 and -L2-R2 are bonded to the two immediately adjacent carbons or a cycloalkylene in which -L1-R1 and -L2-R2 are each bonded to two carbons immediately adjacent to either side of any carbon. This structure can further lower the refractive index and is more effective in improving the efficiency and lifespan of organic light-emitting devices. Here, the cycloalkylene may be in the form of a single ring, a bridge form such as norbonane, or a form in which two or more rings are fused. Additionally, the cycloalkylene may have no double bond or may have one or two double bonds present at any position. Specifically, the cycloalkylene may be substituted or unsubstituted, as defined in Formula 1, and the number of carbon atoms may be within the range of C3 to C50. Specifically, the cycloalkylene may be 1,2-cyclopentylene, 1,2-cyclohexylene, 1,2-cycloheptylene, 1,2-cyclooctylene, 1,2-cyclononylene, 1-2-cyclodecylene , 1,3-cyclopentylene, 1,3-cyclohexylene, 1,3-cycloheptylene, 1,3-cyclooctylene, 1,3-cyclononylene, 1,3-cyclodecylene, 1 , 2-cyclohex-1-ene, 4,5-cyclohex-1-ene, or 2,3-decahydronaphthalene.

In addition, in Formula 1, at least one of -L1-R1 and - L2-R2 may each be independently selected from Structural Formulas B-1 to B-47 shown below.

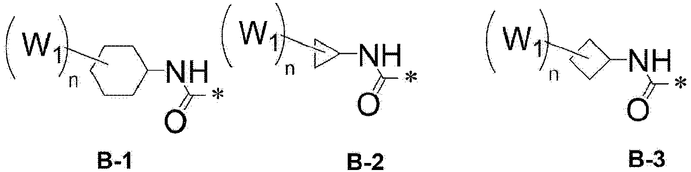

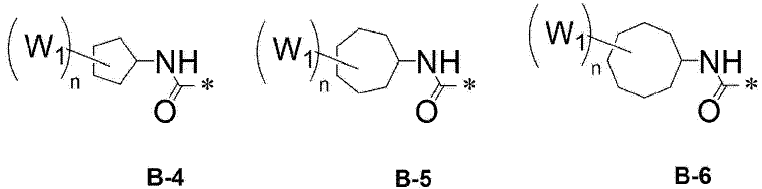

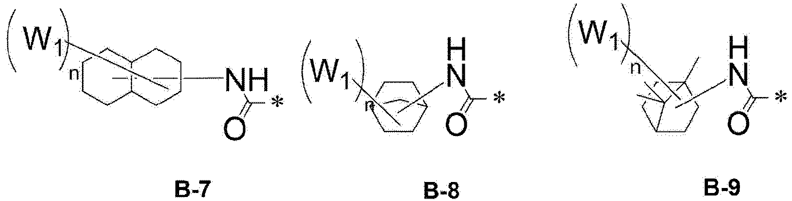

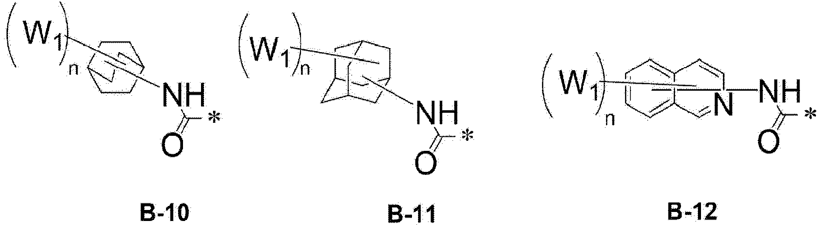

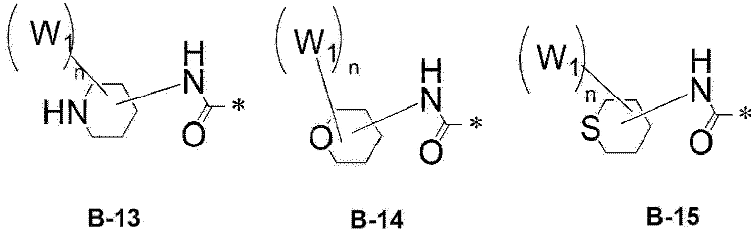

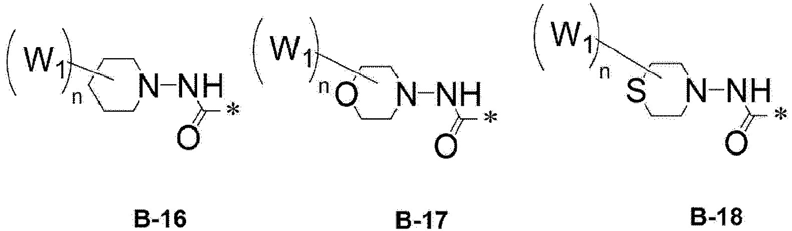

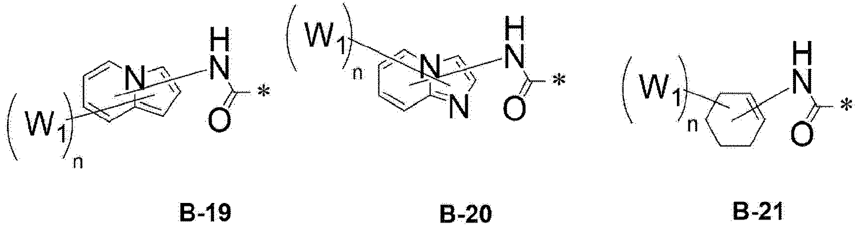

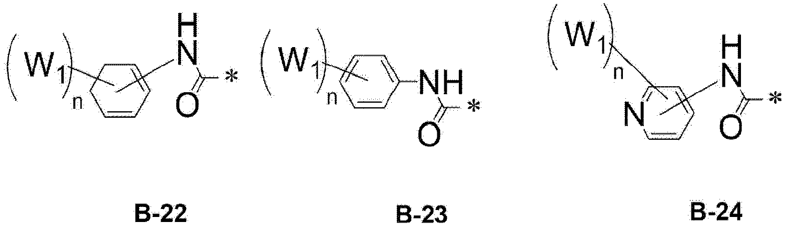

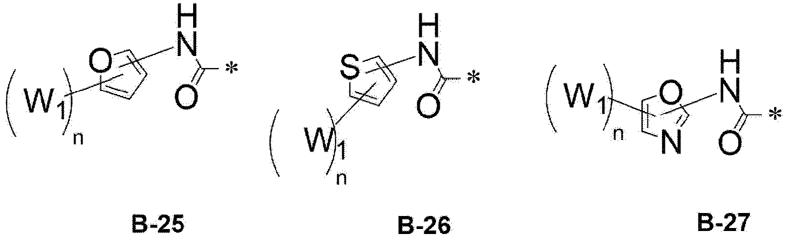

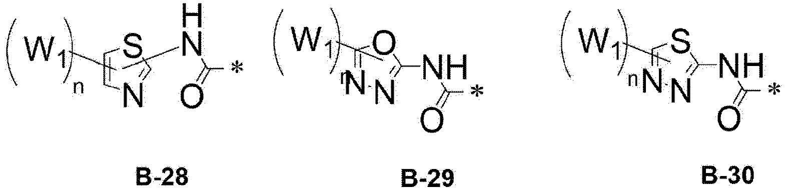

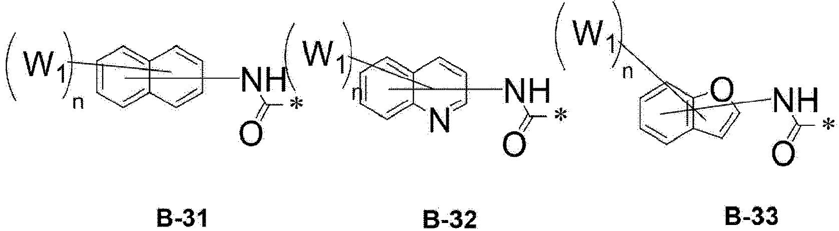

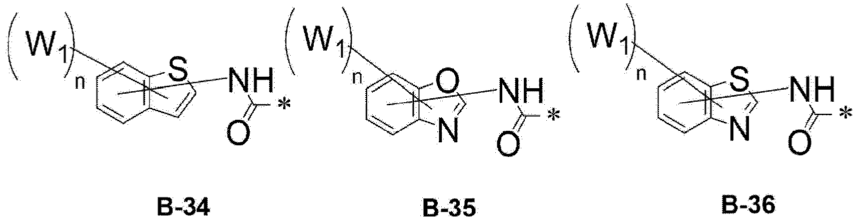

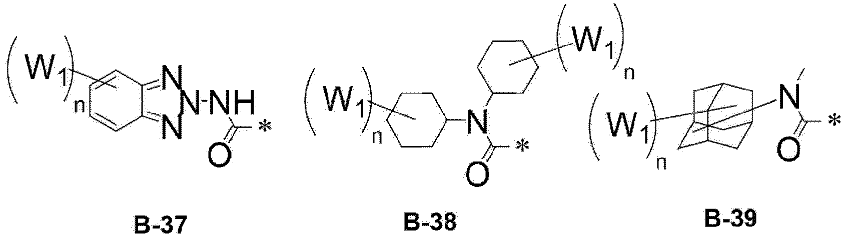

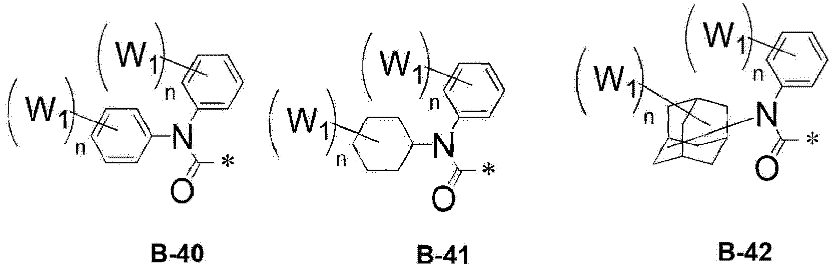

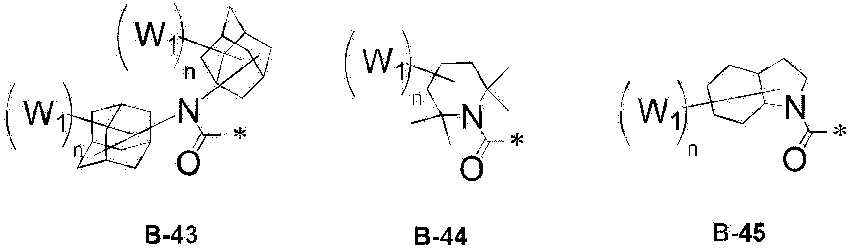

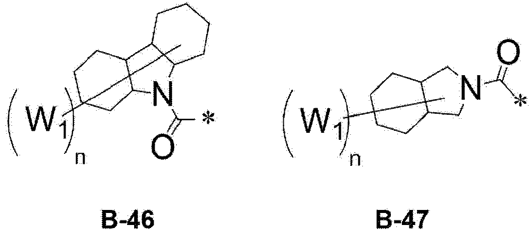

In the structural Formulas, W1 is each independently a methyl group, an ethyl group, a t-butyl group, a cyclohexyl group, an adamantane group, a dihydroamine group, a dimethylamine group, a hydroxy group, a methoxy group, a mercaptan group, methylthio group, a fluoro group, a trifluoromethyl group, or a trimethylsilyl group,
n is each independently an integer in a range of from 0 to 10, specifically an integer in a range of from 0 to 4,
* indicates a bonding site.

In addition, at least one of -L1-R1 and -L2-R2 may each independently have a structural formula in which -NH-CO-* in Structural Formulas B-1 to B-37 is replaced by -Z-* or *-Z-, in which Z is -NH-CS-, -NH-C(=NH)-, -CH2-NH-CO-, -CH2-CO-NH-, -O-NH-CO-, -S-NH-CO-, -CO-NH-CO-, -NH-C(=NMe)-, -NHC(=CHMe)-, -NHCOO-, -NH-, -CO-NH-, -OCONH-, -SCONH-, -CO-CO-NH-, -CH2-, - O-, -S-, -C0-, or "-".

In addition, at least one of -L1-R1 and -L2-R2 may each independently have a structural formula in which N-C0-* structure in Structural Formulas B-38 to B-47 is replaced by N-CS-*, N-C(=NH)-*, N-C(=NMe)-*, NC(=CHMe)-*, NCOO-*, N-*, NCH2-*, NO-*, or NS-*.

In addition, at least one of -L1-R1 and -L2-R2 may each be independently selected from Structural Formulas C-1 through C-12 shown below.

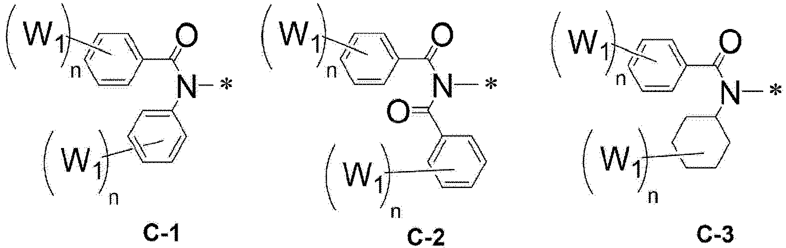

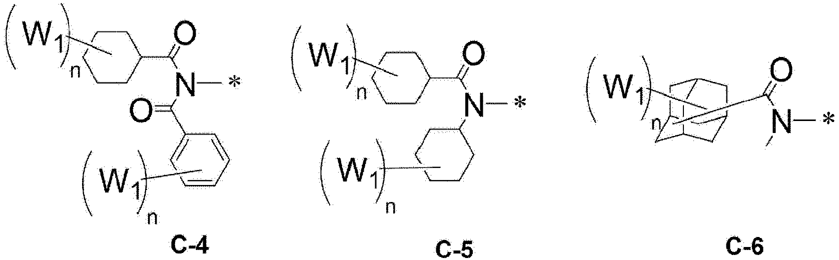

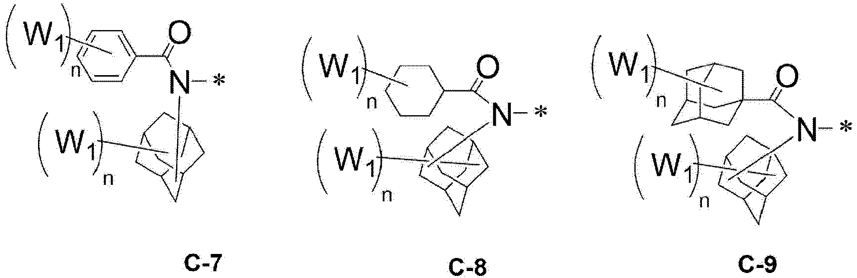

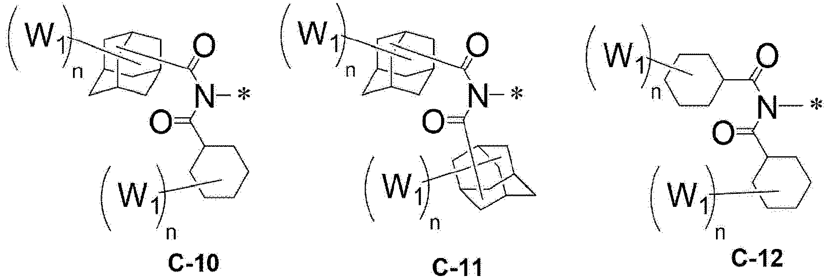

In addition, at least one of -L1-R1 and -L2-R2 may each independently have a structural formula in which -CO-N-* structure in Structural Formulas C-1 through C-12 is replaced by -N-*.

In addition, the capping layer compound represented by Formula 1 may have a low refractive index, such as a refractive index of 1.55 or less at a wavelength of 450 nm, when the refractive index is measured for a thickness range of 20 nm to 100 nm. Specifically, the capping layer compound may have a refractive index of 1.50 or less at a wavelength of 450 nm and more specifically a low refractive index of 1.47 or less at a wavelength of 450 nm.

In addition, the compound of Formula 1 may be a capping layer compound represented by any one of the compounds shown below. The compounds shown below are presented only to help understanding of the present disclosure, and thus the scope of the present disclosure is not limited thereby.

| | | |
|---|---|---|
| 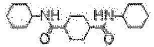 | 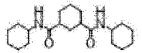 | 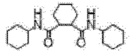 |
| 1 | 2 | 3 |
| 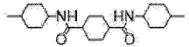 | 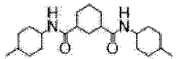 | 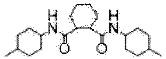 |
| 4 | 5 | 6 |
| 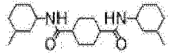 | 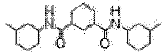 | 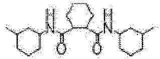 |
| 7 | 8 | 9 |
| 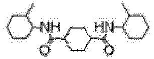 | 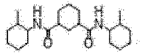 | 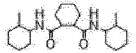 |
| 10 | 11 | 12 |
| 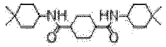 | 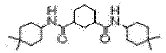 | 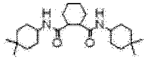 |
| 13 | 14 | 15 |
| 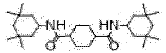 | 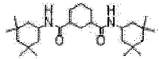 | 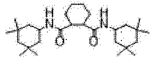 |
| 16 | 17 | 18 |
| 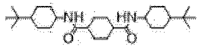 | 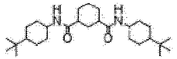 | 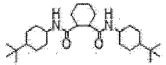 |
| 19 | 20 | 21 |
| 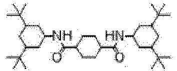 | 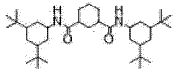 | 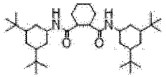 |
| 22 | 23 | 24 |
| 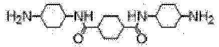 | 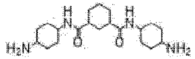 | 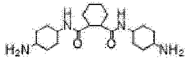 |
| 25 | 26 | 27 |
| 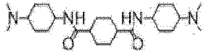 | 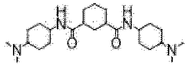 | 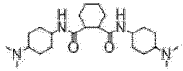 |
| 28 | 29 | 30 |
| 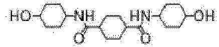 | 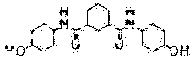 | 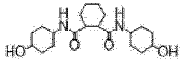 |
| 31 | 32 | 33 |
| 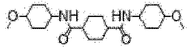 | 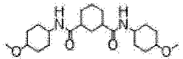 | 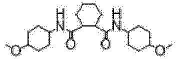 |
| 34 | 35 | 36 |
| 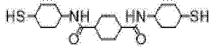 | 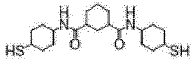 | 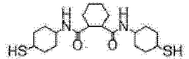 |
| 37 | 38 | 39 |
| 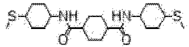 | 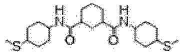 | 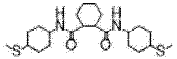 |
| 40 | 41 | 42 |
| 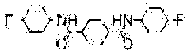 | 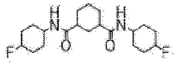 | 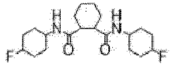 |
| 43 | 44 | 45 |
| 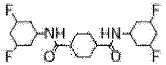 | 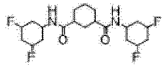 | 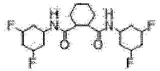 |
| 46 | 47 | 48 |
| 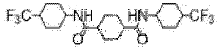 | 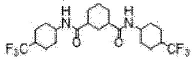 | 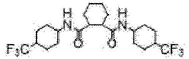 |
| 49 | 50 | 51 |
| 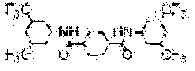 | 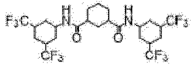 | 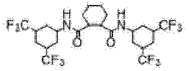 |
| 52 | 33 | 54 |
| 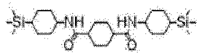 | 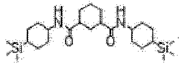 | 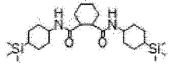 |
| 55 | 56 | 57 |
| 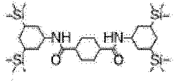 | 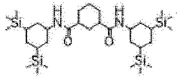 | 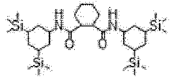 |
| 58 | 59 | 60 |
| 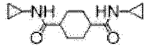 | 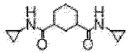 | 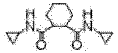 |
| 61 | 62 | 63 |
| 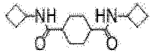 | 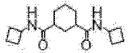 | 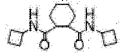 |
| 64 | 65 | 66 |
| 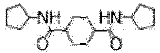 | 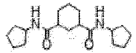 | 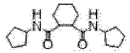 |
| 67 | 68 | 69 |
| 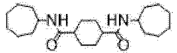 | 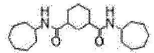 | 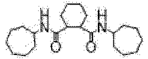 |
| 70 | 71 | 72 |
| 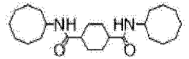 | 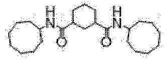 | 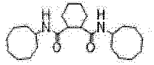 |
| 73 | 74 | 75 |
| 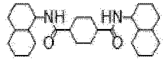 | 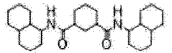 | 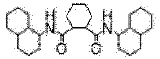 |
| 76 | 77 | 78 |
| 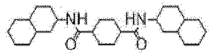 | 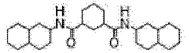 | 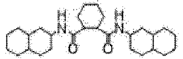 |
| 79 | 80 | 81 |
| 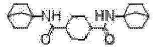 | 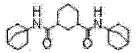 | 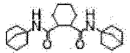 |
| 82 | 83 | 84 |
| 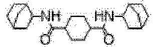 | 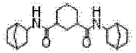 | 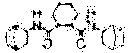 |
| 85 | 86 | 87 |
| 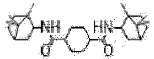 | 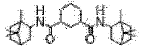 | 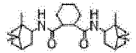 |
| 88 | 89 | 90 |
| 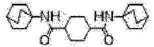 | 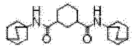 | 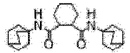 |
| 91 | 92 | 93 |
| 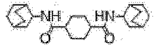 | 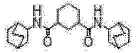 | 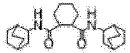 |
| 94 | 95 | 96 |
| 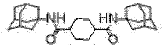 | 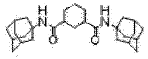 | 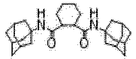 |
| 97 | 98 | 99 |
| 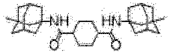 | 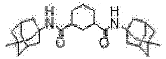 | 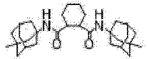 |
| 100 | 101 | 102 |
| 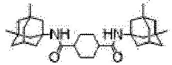 | 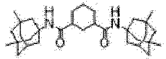 | 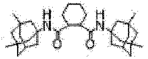 |
| 103 | 104 | 105 |
| 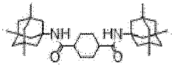 | 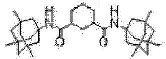 | 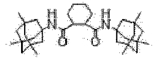 |
| 106 | 107 | 108 |
| 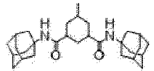 | 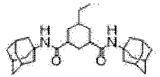 | 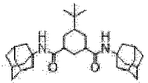 |
| 109 | 110 | 111 |
| 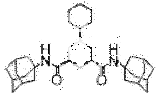 | 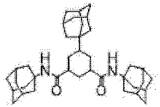 | 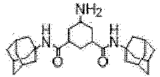 |
| 112 | 113 | 114 |
| 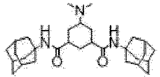 | 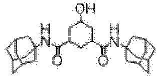 | 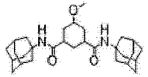 |
| 115 | 116 | 117 |
| 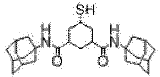 | 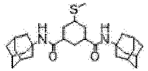 | 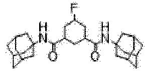 |
| 118 | 119 | 120 |
| 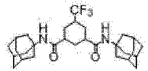 | 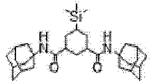 | 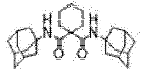 |
| 121 | 122 | 123 |
| 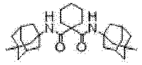 | 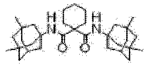 | 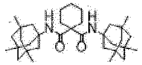 |
| 124 | 125 | 126 |
| 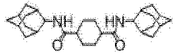 | 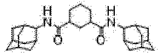 | 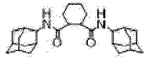 |
| 127 | 128 | 129 |
| 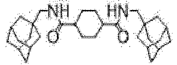 | 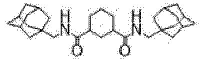 | 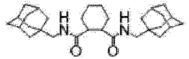 |
| 130 | 131 | 132 |
| 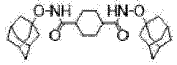 | 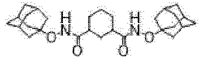 | 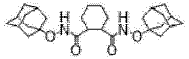 |
| 133 | 134 | 135 |
| 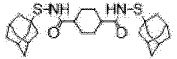 | 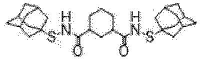 | 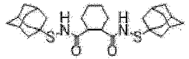 |
| 136 | 137 | 138 |
| 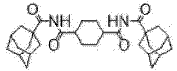 | 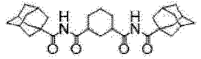 | 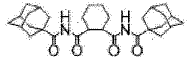 |
| 139 | 140 | 141 |
| 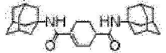 | 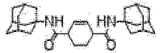 | 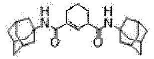 |
| 142 | 143 | 144 |
| 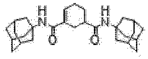 | 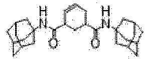 | 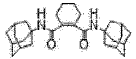 |
| 145 | 146 | 147 |
| 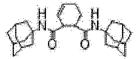 | 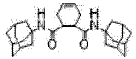 | 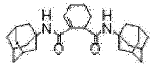 |
| 148 | 149 | 150 |
| 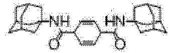 | 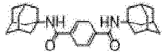 | 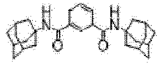 |
| 151 | 152 | 153 |
| 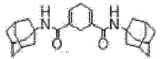 | 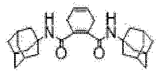 | 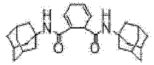 |
| 154 | 155 | 156 |
| 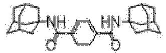 | 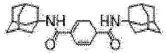 | 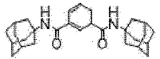 |
| 157 | 158 | 159 |
| 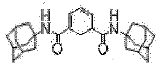 | 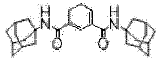 | 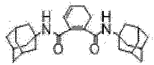 |
| 160 | 161 | 162 |
| 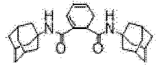 | 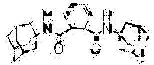 | 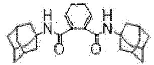 |
| 163 | 164 | 165 |
| 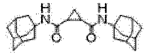 | 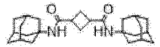 | 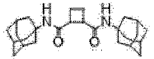 |
| 166 | 167 | 168 |
| 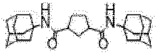 | 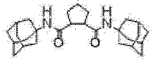 | 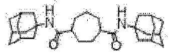 |
| 169 | 170 | 171 |
| 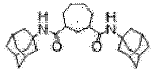 | 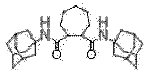 | 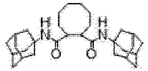 |
| 172 | 173 | 174 |
| 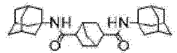 | 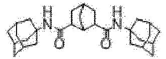 | 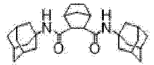 |
| 175 | 176 | 171 |
| 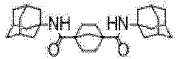 | 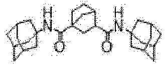 | 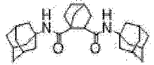 |
| 178 | 179 | 180 |
| 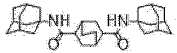 | 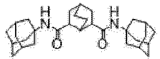 | 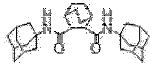 |
| 181 | 182 | 183 |
| 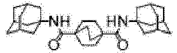 | 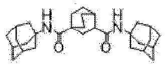 | 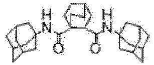 |
| 184 | 185 | 186 |
| 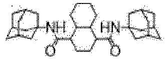 | 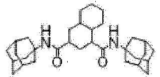 | 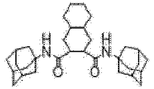 |
| 187 | 188 | 189 |
| 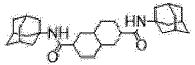 | 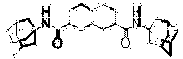 | 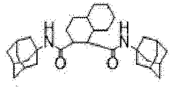 |
| 190 | 191 | 192 |
| 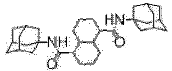 | 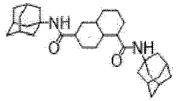 | 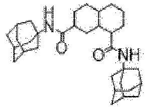 |
| 193 | 194 | 195 |
| 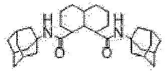 | 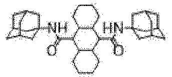 | 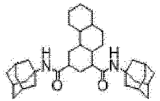 |
| 196 | 197 | 198 |
| 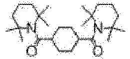 | 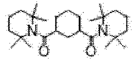 | 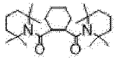 |
| 199 | 200 | 201 |
| 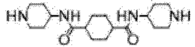 | 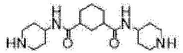 | 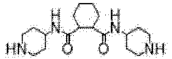 |
| 202 | 203 | 204 |
| 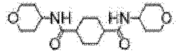 | 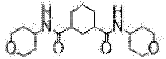 | 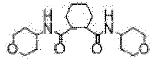 |
| 205 | 206 | 207 |
| 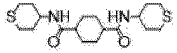 | 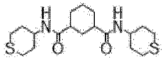 | 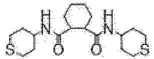 |
| 208 | 209 | 210 |
| 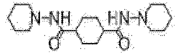 | 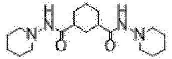 | 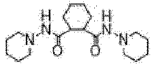 |
| 211 | 212 | 213 |
| 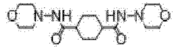 | 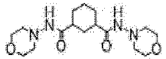 | 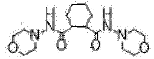 |
| 214 | 215 | 216 |
| 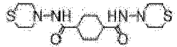 | 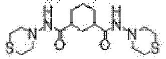 | 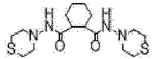 |
| 217 | 218 | 219 |
| 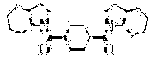 | 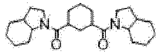 | 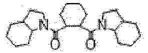 |
| 220 | 221 | 222 |
| 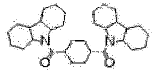 | 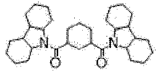 | 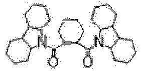 |
| 223 | 224 | 225 |
| 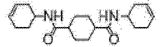 | 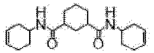 | 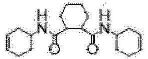 |
| 226 | 227 | 228 |
| 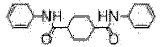 | 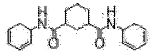 | 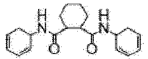 |
| 229 | 230 | 231 |
| 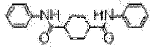 | 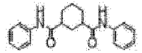 | 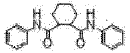 |
| 232 | 233 | 234 |
| 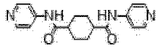 | 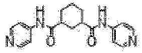 | 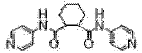 |
| 235 | 236 | 237 |
| 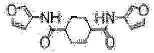 | 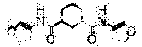 | 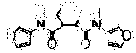 |
| 238 | 239 | 240 |
| 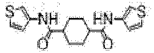 | 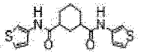 | 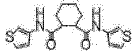 |
| 241 | 242 | 243 |
| 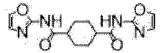 | 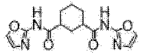 | 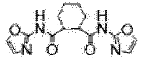 |
| 244 | 245 | 246 |
| 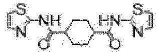 | 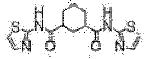 | 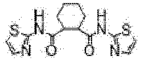 |
| 247 | 248 | 249 |
| 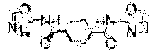 | 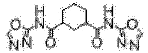 | 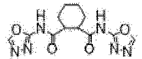 |
| 250 | 251 | 252 |
| 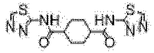 | 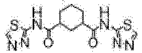 | 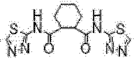 |
| 253 | 254 | 255 |
| 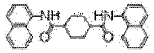 | 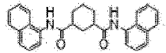 | 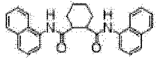 |
| 256 | 257 | 258 |
| 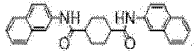 | 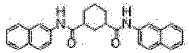 | 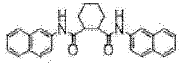 |
| 259 | 260 | 261 |
| 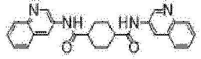 | 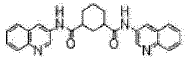 | 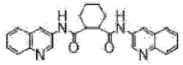 |
| 262 | 263 | 264 |
| 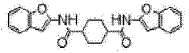 | 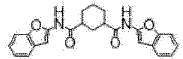 | 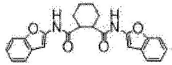 |
| 265 | 266 | 267 |
| 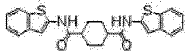 | 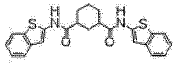 | 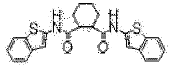 |
| 268 | 269 | 270 |
| 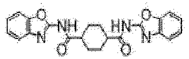 | 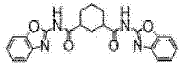 | 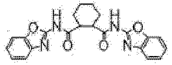 |
| 271 | 212 | 273 |
| 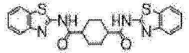 | 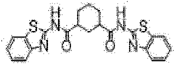 | 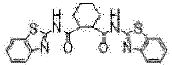 |
| 274 | 275 | 276 |
| 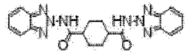 | 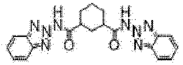 | 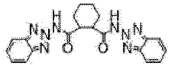 |
| 277 | 278 | 279 |
| 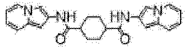 | 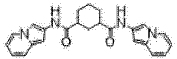 | 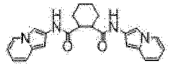 |
| 280 | 281 | 282 |
| 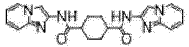 | 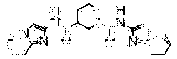 | 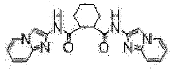 |
| 283 | 284 | 285 |
| 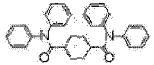 | 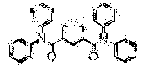 | 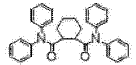 |
| 286 | 287 | 288 |
| 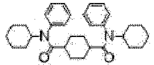 | 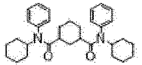 | 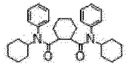 |
| 289 | 290 | 291 |
| 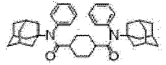 | 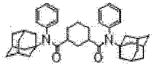 | 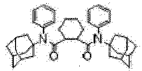 |
| 292 | 293 | 294 |
| 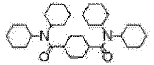 | 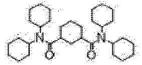 | 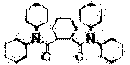 |
| 295 | 296 | 297 |
| 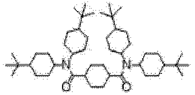 | 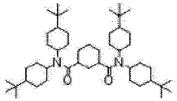 | 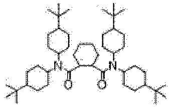 |
| 298 | 299 | 300 |
| 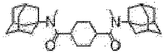 | 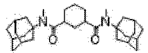 | 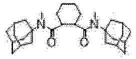 |
| 301 | 302 | 303 |
| 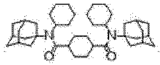 | 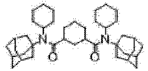 | 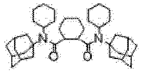 |
| 304 | 305 | 306 |
| 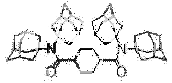 | 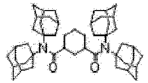 | 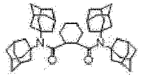 |
| 307 | 308 | 309 |
| 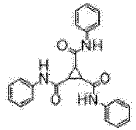 | 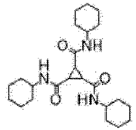 | 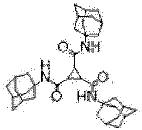 |
| 310 | 311 | 312 |
| 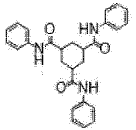 | 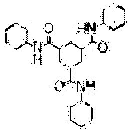 | 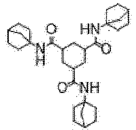 |
| 313 | 314 | 315 |
| 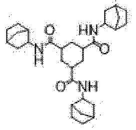 | 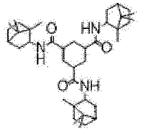 | 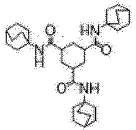 |
| 316 | 317 | 318 |
| 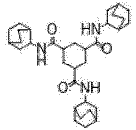 | 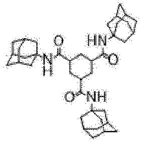 | 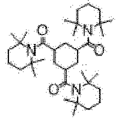 |
| 319 | 320 | 321 |
| 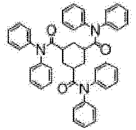 | 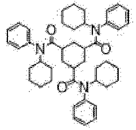 | 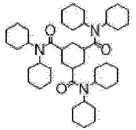 |
| 322 | 323 | 324 |
| 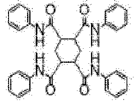 | 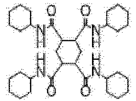 | 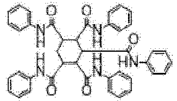 |
| 325 | 326 | 327 |
| 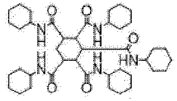 | 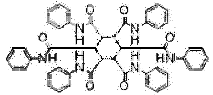 | 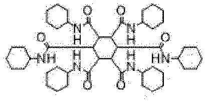 |
| 328 | 329 | 330 |
| 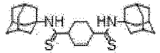 | 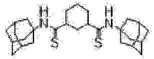 | 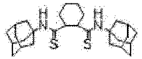 |
| 331 | 332 | 333 |
| 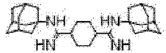 | 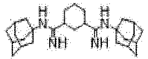 | 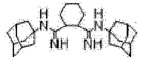 |
| 334 | 335 | 336 |
| 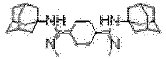 | 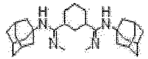 | 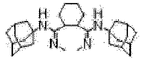 |
| 337 | 338 | 339 |
| 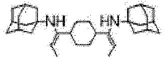 | 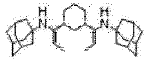 | 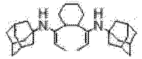 |
| 340 | 341 | 342 |
| 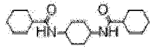 | 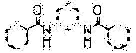 | 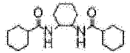 |
| 343 | 344 | 345 |
| 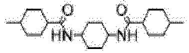 | 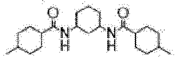 | 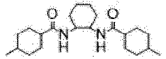 |
| 346 | 347 | 348 |
| 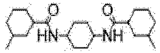 | 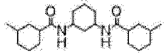 | 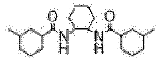 |
| 349 | 350 | 351 |
| 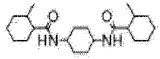 | 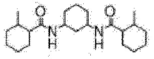 | 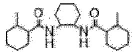 |
| 352 | 353 | 354 |
| 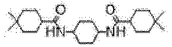 | 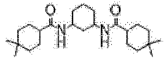 | 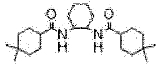 |
| 355 | 356 | 357 |
| 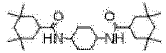 | 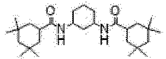 | 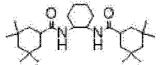 |
| 358 | 359 | 360 |
| 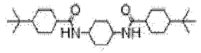 | 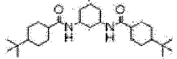 | 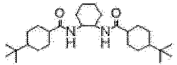 |
| 361 | 362 | 363 |
| 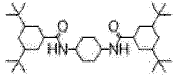 | 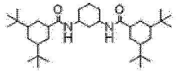 | 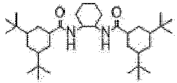 |
| 364 | 365 | 366 |
| 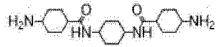 | 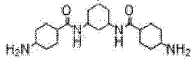 | 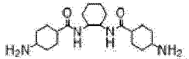 |
| 367 | 368 | 369 |
| 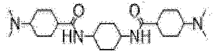 | 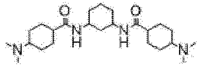 | 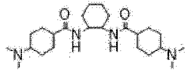 |
| 370 | 371 | 372 |
| 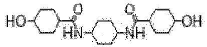 | 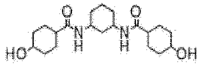 | 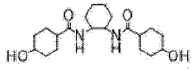 |
| 373 | 374 | 375 |
| 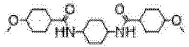 | 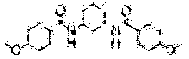 | 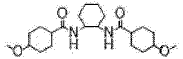 |
| 376 | 377 | 378 |
| 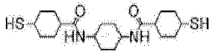 | 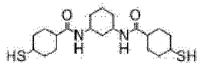 | 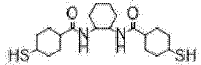 |
| 379 | 380 | 381 |
| 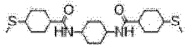 | 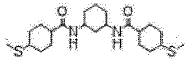 | 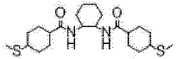 |
| 382 | 383 | 384 |
| 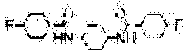 | 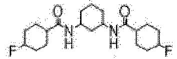 | 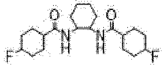 |
| 385 | 386 | 387 |
| 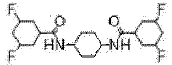 | 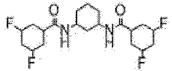 | 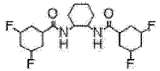 |
| 388 | 389 | 390 |
| 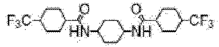 | 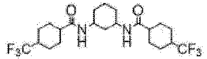 | 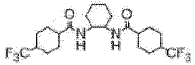 |
| 391 | 392 | 393 |
| 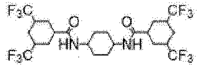 | 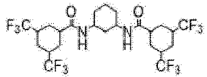 | 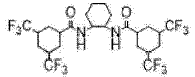 |
| 394 | 395 | 396 |
| 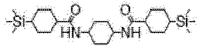 | 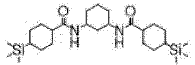 | 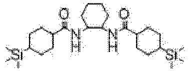 |
| 397 | 398 | 399 |
| 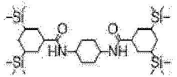 | 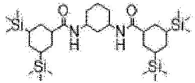 | 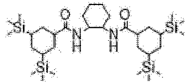 |
| 400 | 401 | 402 |
| 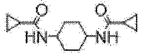 | 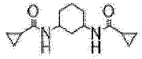 | 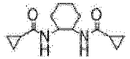 |
| 403 | 404 | 405 |
| 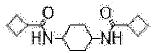 | 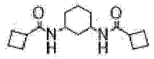 | 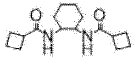 |
| 406 | 407 | 408 |
| 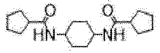 | 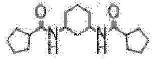 | 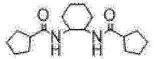 |
| 409 | 410 | 411 |
| 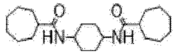 | 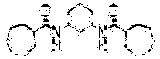 | 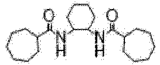 |
| 412 | 413 | 414 |
| 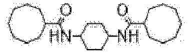 | 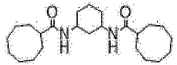 | 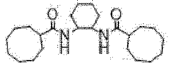 |
| 415 | 416 | 417 |
| 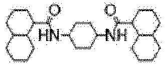 | 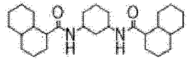 | 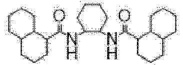 |
| 418 | 419 | 420 |
| 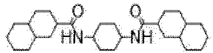 | 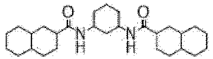 | 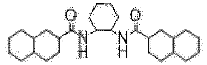 |
| 421 | 422 | 423 |
| 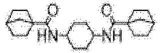 | 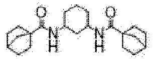 | 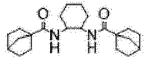 |
| 424 | 425 | 426 |
| 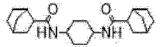 | 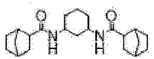 | 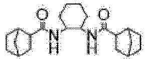 |
| 427 | 428 | 429 |
| 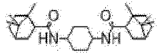 | 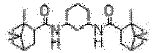 | 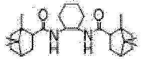 |
| 430 | 431 | 432 |
| 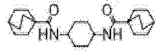 | 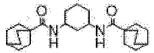 | 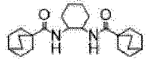 |
| 433 | 434 | 435 |
| 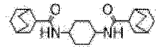 | 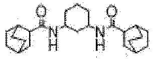 | 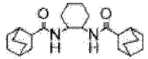 |
| 436 | 437 | 438 |
| 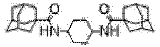 | 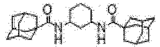 | 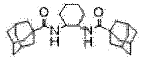 |
| 439 | 440 | 441 |
| 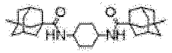 | 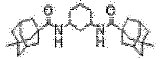 | 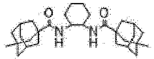 |
| 442 | 443 | 444 |
| 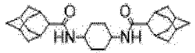 | 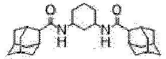 | 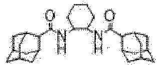 |
| 445 | 446 | 447 |
| 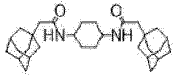 | 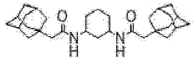 | 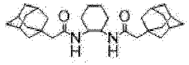 |
| 448 | 449 | 450 |
| 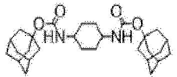 | 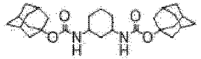 | 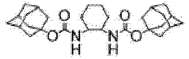 |
| 451 | 452 | 453 |
| 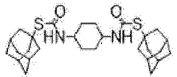 | 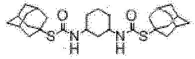 | 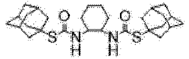 |
| 454 | 455 | 456 |
| 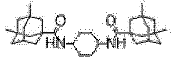 | 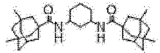 | 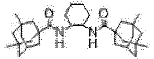 |
| 457 | 458 | 459 |
| 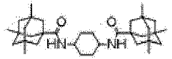 | 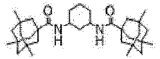 | 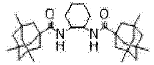 |
| 460 | 461 | 462 |
| 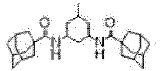 | 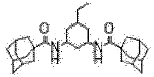 | 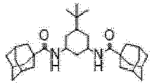 |
| 463 | 464 | 465 |
| 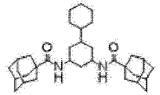 | 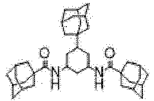 | 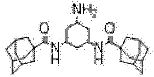 |
| 466 | 467 | 468 |
| 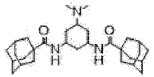 | 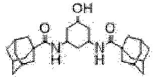 | 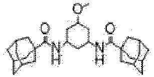 |
| 469 | 470 | 471 |
| 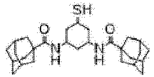 | 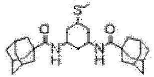 | 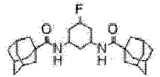 |
| 472 | 473 | 474 |
| 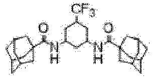 | 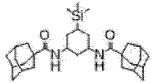 | 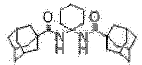 |
| 475 | 476 | 477 |
| 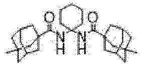 | 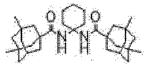 | 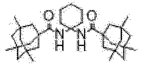 |
| 478 | 479 | 480 |
| 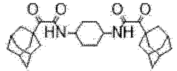 | 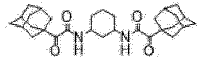 | 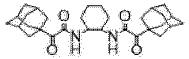 |
| 481 | 482 | 483 |
| 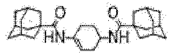 | 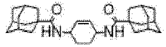 | 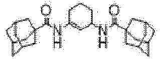 |
| 484 | 485 | 486 |
| 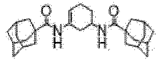 | 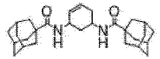 | 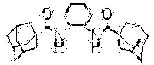 |
| 487 | 488 | 489 |
| 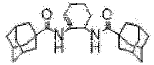 | 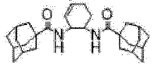 | 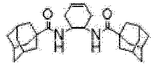 |
| 490 | 491 | 492 |
| 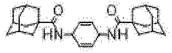 | 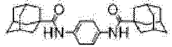 | 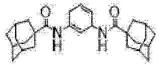 |
| 493 | 494 | 495 |
| 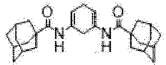 | 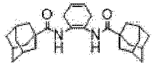 | 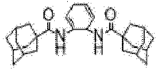 |
| 496 | 497 | 498 |
| 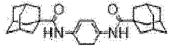 | 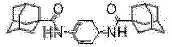 | 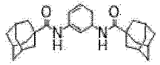 |
| 499 | 500 | 501 |
| 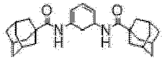 | 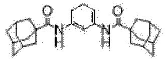 | 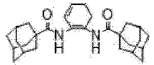 |
| 502 | 503 | 504 |
| 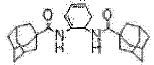 | 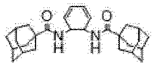 | 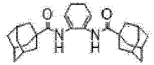 |
| 505 | 506 | 507 |
| 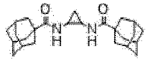 | 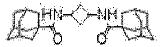 | 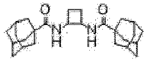 |
| 508 | 509 | 510 |
| 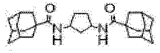 | 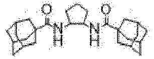 | 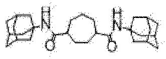 |
| 511 | 512 | 513 |
| 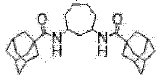 | 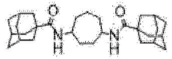 | 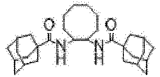 |
| 514 | 515 | 516 |
| 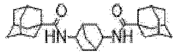 | 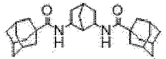 | 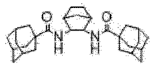 |
| 517 | 518 | 519 |
| 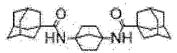 | 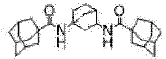 | 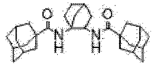 |
| 520 | 521 | 522 |
| 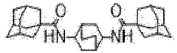 | 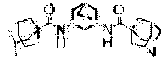 | 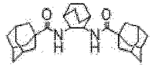 |
| 523 | 524 | 525 |
| 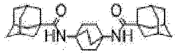 | 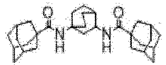 | 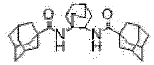 |
| 526 | 527 | 528 |
| 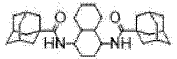 | 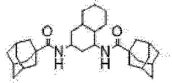 | 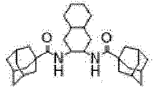 |
| 529 | 530 | 531 |
| 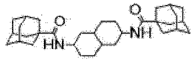 | 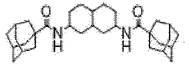 | 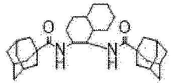 |
| 532 | 533 | 534 |
| 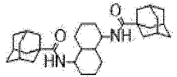 | 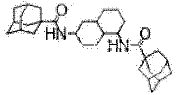 | 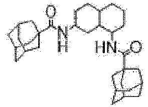 |
| 535 | 536 | 537 |
| 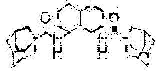 | 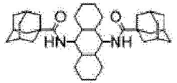 | 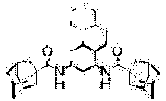 |
| 538 | 539 | 540 |
| 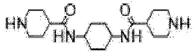 | 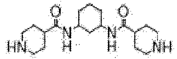 | 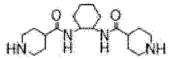 |
| 541 | 542 | 543 |
| 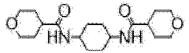 | 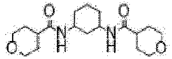 | 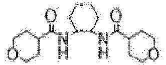 |
| 544 | 545 | 546 |
| 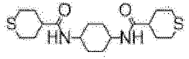 | 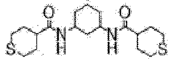 | 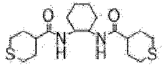 |
| 547 | 548 | 549 |
| 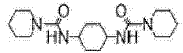 | 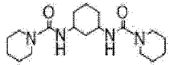 | 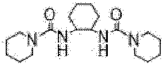 |
| 550 | 551 | 552 |
| 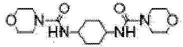 | 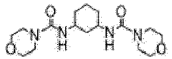 | 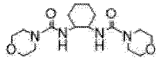 |
| 553 | 554 | 555 |
| 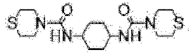 | 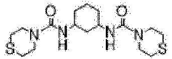 | 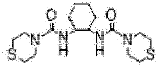 |
| 556 | 557 | 558 |
| 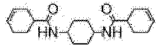 | 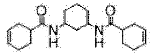 | 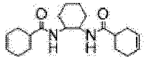 |
| 559 | 560 | 561 |
| 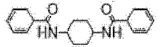 | 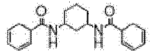 | 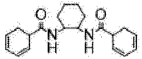 |
| 562 | 563 | 564 |
| 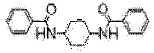 | 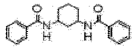 | 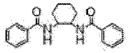 |
| 565 | 566 | 567 |
| 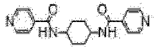 | 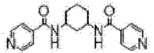 | 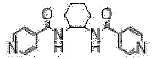 |
| 568 | 569 | 570 |
| 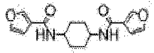 | 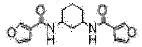 | 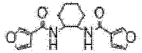 |
| 571 | 572 | 573 |
| 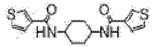 | 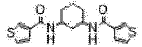 | 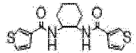 |
| 574 | 575 | 576 |
| 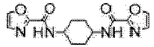 | 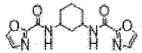 | 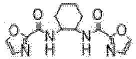 |
| 577 | 578 | 579 |
| 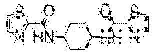 | 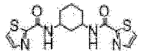 | 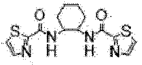 |
| 580 | 581 | 582 |
| 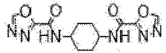 | 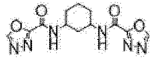 | 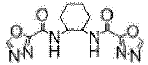 |
| 583 | 584 | 585 |
| 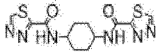 | 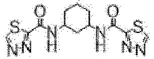 | 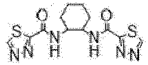 |
| 586 | 587 | 588 |
| 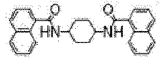 | 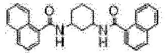 | 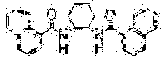 |
| 589 | 590 | 591 |
| 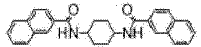 | 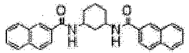 | 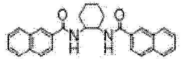 |
| 592 | 593 | 594 |
| 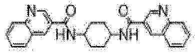 | 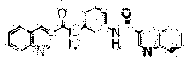 | 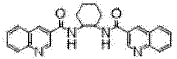 |
| 595 | 596 | 597 |
| 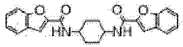 | 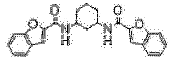 | 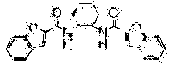 |
| 598 | 599 | 600 |
| 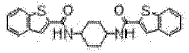 | 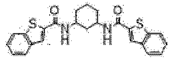 | 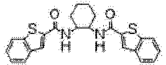 |
| 601 | 602 | 603 |
| 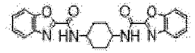 | 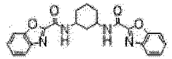 | 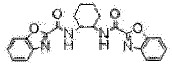 |
| 604 | 605 | 606 |
| 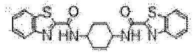 | 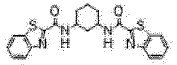 | 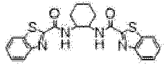 |
| 607 | 608 | 609 |
| 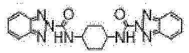 | 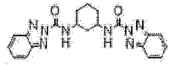 | 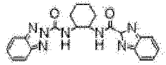 |
| 610 | 611 | 612 |
| 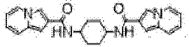 | 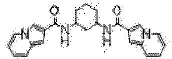 | 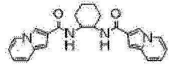 |
| 613 | 614 | 615 |
| 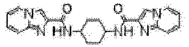 | 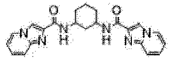 | 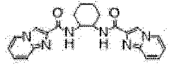 |
| 616 | 617 | 618 |
| 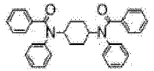 | 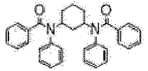 | 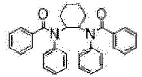 |
| 619 | 620 | 621 |
| 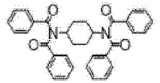 | 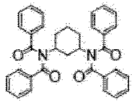 | 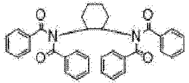 |
| 622 | 623 | 624 |
| 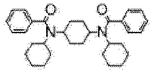 | 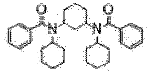 | 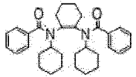 |
| 625 | 626 | 627 |
| 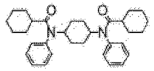 | 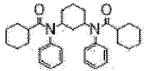 | 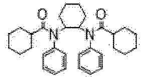 |
| 628 | 629 | 630 |
| 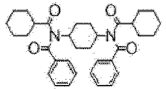 | 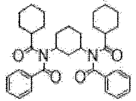 | 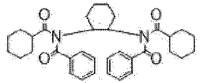 |
| 631 | 632 | 633 |
| 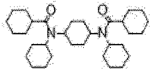 | 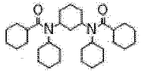 | 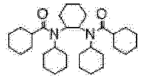 |
| 634 | 635 | 636 |
| 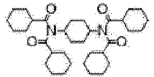 | 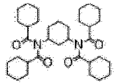 | 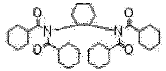 |
| 637 | 638 | 639 |
| 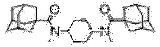 | 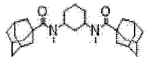 | 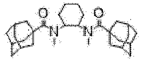 |
| 640 | 641 | 642 |
| 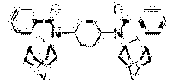 | 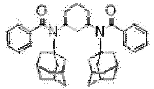 | 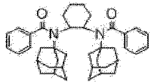 |
| 643 | 644 | 645 |
| 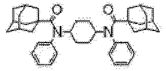 | 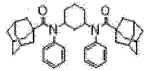 | 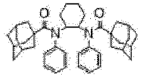 |
| 646 | 647 | 648 |
| 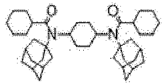 | 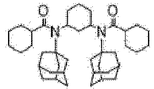 | 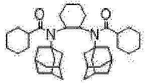 |
| 649 | 650 | 651 |
| 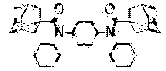 | 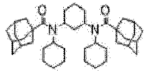 | 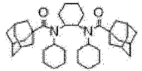 |
| 652 | 653 | 654 |
| 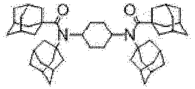 | 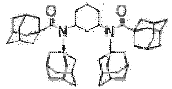 | 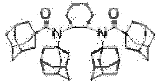 |
| 655 | 656 | 657 |
| 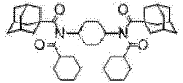 | 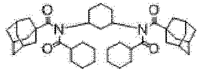 | 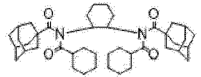 |
| 658 | 659 | 660 |
| 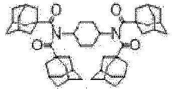 | 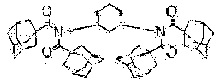 | 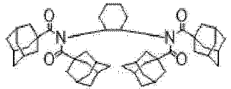 |
| 661 | 662 | 663 |
| 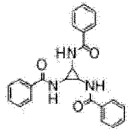 | 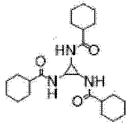 | 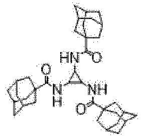 |
| 664 | 665 | 666 |
| 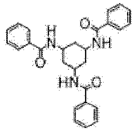 | 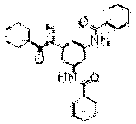 | 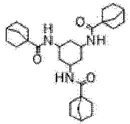 |
| 667 | 668 | 669 |
| 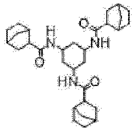 | 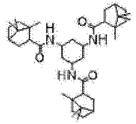 | 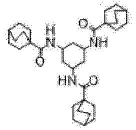 |
| 670 | 671 | 672 |
| 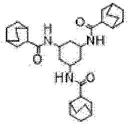 | 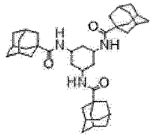 | 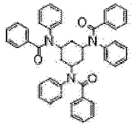 |
| 673 | 674 | 675 |
| 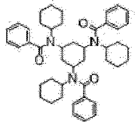 | 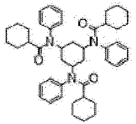 | 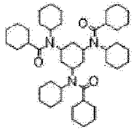 |
| 676 | 677 | 678 |
| 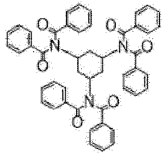 | 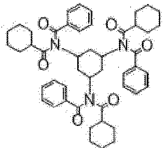 | 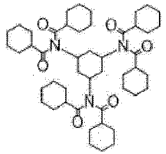 |
| 679 | 680 | 681 |
| 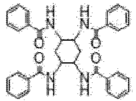 | 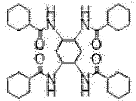 | 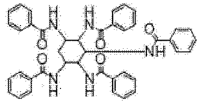 |
| 682 | 683 | 684 |
| 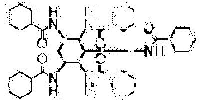 | 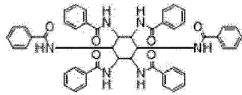 | 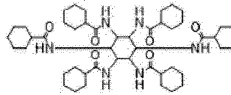 > |
| 685 | 686 | 687 |
| 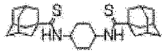 | 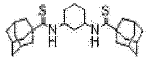 | 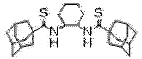 |
| 688 | 689 | 690 |
| 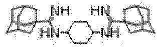 | 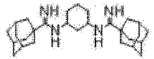 | 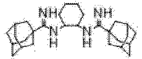 |
| 691 | 692 | 693 |
| 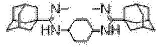 | 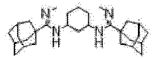 | 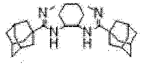 |
| 694 | 695 | 696 |
| 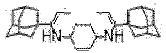 | 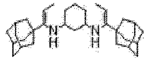 | 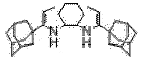 |
| 697 | 698 | 699 |
| 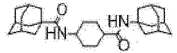 | 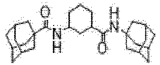 | 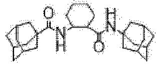 |
| 700 | 701 | 702 |
| 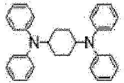 | 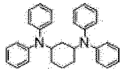 | 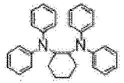 |
| 703 | 704 | 705 |
| 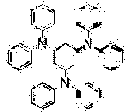 | 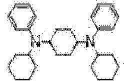 | 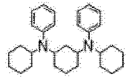 |
| 706 | 707 | 708 |
| 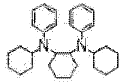 | 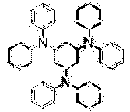 | 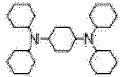 |
| 709 | 710 | 711 |
| 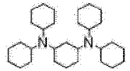 | 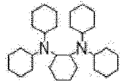 | 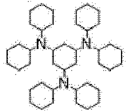 |
| 712 | 713 | 714 |
| 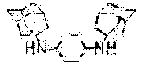 | 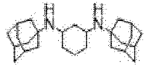 | 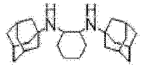 |
| 715 | 716 | 717 |
| 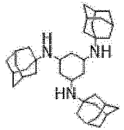 | 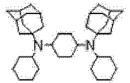 | 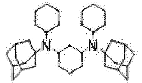 |
| 718 | 719 | 720 |
| 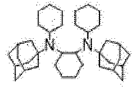 | 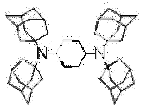 | 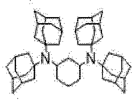 |
| 721 | 722 | 723 |
| 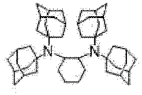 | 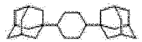 | 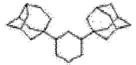 |
| 724 | 725 | 726 |
| 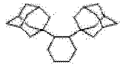 | 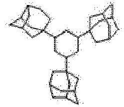 | 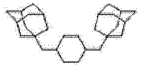 |
| 727 | 728 | 729 |
| 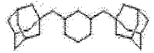 | 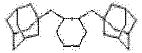 | 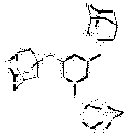 |
| 730 | 731 | 732 |
| 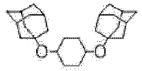 | 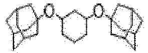 | 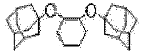 |
| 733 | 734 | 735 |
| 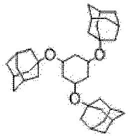 | 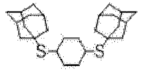 | 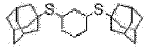 |
| 736 | 737 | 738 |
| 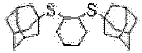 | 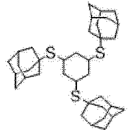 | 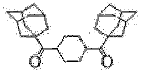 |
| 739 | 740 | 741 |
| 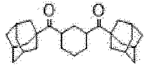 | 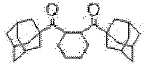 | 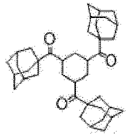 |
| 742 | 743 | 744 |

One example of the compound of the present disclosure can be synthesized by schematic reaction formulas shown below.

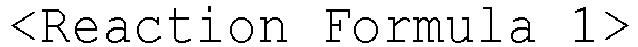

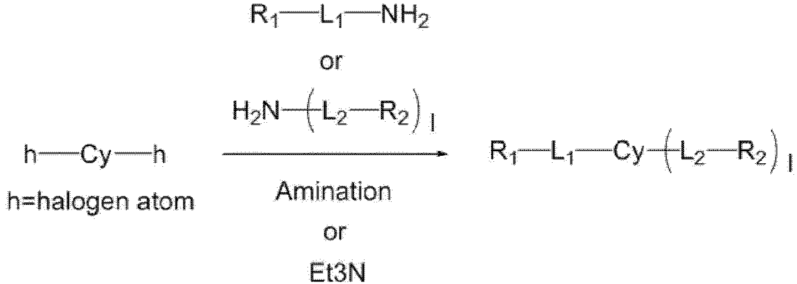

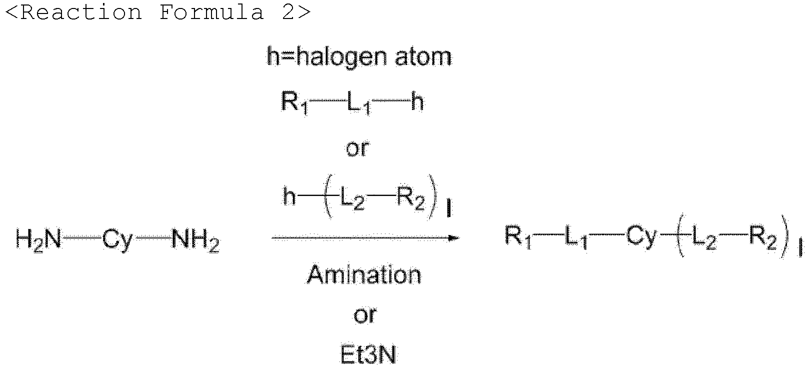

In another aspect of the present disclosure, there is provided an organic light-emitting device including a capping layer containing the capping layer compound described above.

Hereinafter, an organic light-emitting device according to one embodiment of the present disclosure will be described in detail.

According to one embodiment of the present disclosure, the organic light-emitting device includes a first electrode, a second electrode, one or more organic layers interposed between the first electrode and the second electrode, and a capping layer, in which the capping layer is disposed on the outer surface of either the first electrode or the second electrode.

Specifically, of the both surfaces of each of the first and second electrodes, a surface adjacent to the organic layer interposed between the first electrode and the second electrode is referred to as an inner surface, and a surface not adjacent to the organic material layer is referred to as an outer surface. That is, when the capping layer is disposed on the outer surface of the first electrode, the first electrode is interposed between the capping layer and the organic layer. When the capping layer is disposed on the outer surface of the second electrode, the second electrode is interposed between the capping layer and the organic layer.

According to one embodiment of the present disclosure, in the organic light-emitting device, one or more organic layers may be interposed between the first electrode and the second electrode or may be disposed on the outer surface of at least one of the first and second electrodes. That is, the capping layer may be formed on the outer surface of each of the first and second electrodes or may be formed on the outer surface of either one of the first and second electrodes. The capping layer may contain the capping layer compound according to the present disclosure. The capping layer may contain only one compound or two or more compounds, selected from the capping layer compounds of the present disclosure. The capping layer may contain one or more of the capping layer compounds and other known compounds.

The capping layer may have a thickness of 100 to 3000 Å.

On the other hand, the capping layer may have a composite capping layer structure in which a first capping layer having a relatively low refractive index and a second capping layer having a higher refractive index than the first capping layer are laminated. In this case, the compound for a capping layer, according to the present disclosure, may be included in the first capping layer. The stacking order of the first capping layer and the second capping layer is not limited. The first capping layer may be disposed on the outer side of the second capping layer. Alternatively, the second capping layer may be disposed on the outer side of the first capping layer. In a specific example, the second capping layer may be interposed between the first capping layer and the first or second electrode. Specifically, the second capping layer may be in contact with the first capping layer and the first electrode or in contact with the first capping layer and the second electrode.

In addition, the capping layer may be a multilayer structure in which a plurality of first capping layers and a plurality of second capping layers are stacked. The first capping layers and the second capping layers may be alternately stacked. That is, the stacking order is not limited as described above. The first capping layer may be disposed on the outer side of the second capping layer. Conversely, the second capping layer may be disposed on the outer side of the first capping layer.

In addition, the first capping layer may have a refractive index of 1.55 or less, specifically a refractive index of 1.50 or less, and more specifically a refractive index of 1.47 or less, at a wavelength of 450 nm. The second capping layer may have a refractive index of 2.10 or more, specifically a refractive index of 2.25 or more, and more specifically 2.30 or more, at a wavelength of 450 nm. The difference in refractive index between the first capping layer and the second capping layer at a wavelength of 450 nm may be in the range of 0.2 to 1.2 and more specifically 0.4 to 1.2. When the refractive index difference is less than 0.2 or greater than 1.2, the light extraction efficiency will be reduced.

The total thickness of the first capping layer may be in the range of from 50 Å to 2000 Å, and the total thickness of the second capping layer may be in the range of 50 Å to 2000 Å.

On the other hand, the capping layer may have a refractive index gradient. The refractive index gradient may have a descending profile in which the refractive index gradually decreases in an outward direction or an ascending profile in which the refractive index gradually increases in the outward direction. To this end, the refractive index gradient in the capping layer may be implemented by gradually varying the concentration of the capping layer compound according to the present disclosure during the formation of the capping layer.

On the other hand, the organic layers may include a hole transport layer, a light-emitting layer, and an electron transport layer that that constitute a light-emitting unit but may not be limited thereto.

More specifically, the organic light-emitting device according to one embodiment of the present disclosure includes one or more organic layers between the first electrode (anode) and the second electrode (cathode), in which the organic layers are layers selected from a hole injection layer (HIL), a hole transport layer (HTL), and a light-emitting layer (HTL), an electron transport layer (ETL), and an electron injection layer (EIL). Optionally, a hole-blocking layer (HBL, not shown) or an electron transport auxiliary layer may be further disposed between the light-emitting layer (EML) and the electron transport layer (ETL), and an electron-blocking layer (EBL, not shown) or a light-emitting auxiliary layer may be disposed between the hole transport layer (HTL) and the light-emitting layer (EML).

FIG. 1 is a cross-sectional view schematically illustrating the construction of an organic light-emitting device according to one embodiment of the present disclosure. The organic light-emitting device according to one embodiment of the present disclosure may be manufactured to have a structure illustrated in FIG. 1.

Referring to FIG. 1, the organic light-emitting device includes a substrate 100, a first electrode 1000, a hole injection layer 200, a hole transport layer 300, a light-emitting layer 400, an electron transport layer 500, an electron injection layer 600, a second electrode 2000, and a capping layer 3000 that are stacked in this order. Although not shown in the illustration, the capping layer 3000 may be a laminate in which the first capping layer and the second capping layer are stacked on another, as described above. The capping layer 3000 may be a laminate that includes not only the first and second capping layers and a third capping layer having a different refractive index from each of the first and second capping layers. In addition, the capping layer may have a refractive index gradient. The refractive index gradient may have a descending profile in which the refractive index gradually decreases in an outward direction or an ascending profile in which the refractive index gradually increases in the outward direction.

Here, as the substrate 100, a substrate that is commonly used for organic light-emitting devices may be used. Specifically, a transparent glass substrate or a flexible plastic substrate excellent in mechanical strength, thermal stability, transparency, surface flatness, easy handling, and waterproofness may be used.

In the organic light-emitting device, the first electrode 1000 is used as a hole injection electrode for injecting holes.

The first electrode 1000 is made of a material having a low work function to enable hole injection. Specifically, the first electrode 1000 is made of a transparent material such as indium tin oxide (ITO), indium zinc oxide (IZO), or graphene.

The hole injection layer 200 may be formed by depositing a hole injection material on the first electrode 1000 by a method such as a vacuum deposition method, a spin coating method, a casting method, Langmuir-Blodgett (LB), or the like. In the case of using a vacuum deposition method to form the hole injection layer 200, the deposition conditions vary depending on the compound used as the material of the hole injection layer 200, the structure and thermal characteristics of the desired hole injection layer 200, and the like. The conditions are appropriately set to fall within a temperature range of 50°C to 500°C, a vacuum degree range of 10-8 to 10-3 torr, a deposition rate range of 0.01 to 100 Å/sec, and a layer thickness range of 10 Å to 5 um. A charge generating layer may be optionally deposited on the surface of the hole injection layer 200 if necessary. A conventional material may be used as the material of the charge generation layer. For example, HATCN may be used.

The hole transport layer 300 may be formed by depositing a hole transport material on the hole injection layer 200 by a method such as a vacuum deposition method, a spin coating method, a casting method, or LB. In the case of forming the hole transport layer 300 by the vacuum deposition method, the deposition conditions vary depending on the compound used. However, the conditions may be selected from the same ranges described in connection with the hole injection layer 200. The hole transport layer 300 may be formed using a known compound. The hole transport layer 300 may be a single layer structure or a multilayer structure. Although not illustrated in FIG. 1, a light-emitting auxiliary layer may be additionally formed on the hole transport layer 300.

The light-emitting layer 400 may be formed by depositing a light-emitting material on the hole transport layer 300 or the light-emitting auxiliary layer by a method such as a vacuum deposition method, a spin coating method, a casting method, or LB. In the case of using a vacuum deposition method to form the light-emitting layer 400, the deposition conditions vary depending on the compound used. However, the conditions may be selected from the same ranges as in the deposition conditions of the hole injection layer 200. As the light-emitting material, a known compound may be used as a host or a dopant. The dopant is not limited to a specific substance. The light-emitting layer may be formed using a phosphorescent or fluorescent dopant. For example, BD142 (N6,N12-bis(3,4-dimethylphenyl)-N6,N12-dimethylchrysene-6,12-diamine) can be used as the fluorescent dopant, and the green phosphorescent dopant "Ir(ppy)3 (tris(2-phenylpyridine)iridium)", the blue phosphorescent dopant "F2Irpic (iridium(III) bis[4,6-difluorophenyl-pyridinato-N,C2']picolinate)", and the red phosphorescent dopant "RD61" available from UDC Corporation can be vacuum-deposited (doped) together as the phosphorescent dopants. The doping concentration of the dopant is not particularly limited, but it is preferred that the doping concentration of the dopant may be in the range of from 0.01 to 15 parts by weight relative to 100 parts by weight of the host. When the content of the dopant is less than 0.01 wt%, since the amount of the dopant is insufficient, the coloration is not properly achieved. When the content of the dopant exceeds 15 wt%, the efficiency is sharply reduced due to the concentration quenching phenomenon.

Here, in the case where the phosphorescent dopant and the light-emitting material are used together, a hole-blocking material (HBL) may be deposited on the light-emitting layer 400 by a vacuum deposition method or a spin coating method to prevent triplet excitons or holes from diffusing into the electron transport layer 500. The material that can be used as the hole-blocking material is not particularly limited, and an arbitrary existing material may be selected and used. Examples of the hole blocking material include oxadiazole derivatives, triazole derivatives, phenanthroline derivatives, and hole blocking materials described in Japanese Patent Application Publication No. H11-329734(A1). Representatively, Balq(bis(8-hydroxy-2-methylquinolinolato)-aluminum biphenoxide), and phenanthrolines-based compounds (for example, Bathocuproine (BCP) available from UDC) may be used. The light-emitting layer 400 used in the embodiment of the present disclosure may include one or more blue light-emitting layer.

The electron transport layer 500 is formed on the light-emitting layer 400 by a vacuum deposition method, a spin coating method, a casting method, or the like. The deposition conditions for the electron transport layer 500 vary depending on the compound used. However, the conditions may be selected from almost the same ranges as in the deposition conditions of the hole injection layer 200. For example, quinoline derivatives, in particular tris(8-quinolinolato)aluminum (Alq3), or 6,6'-(3,4-dimethyl-1,1-dimethyl-1H-silol-2,5-diyl)di-2,2'-bipyridine)(ET4), can be commonly used.

The electron injection layer 600 is formed by depositing an electron injection material on the electron transport 500 by a vacuum deposition method, a spin coating method, a casting method, or the like. As the material for the electron injection layer, LiF, NaCl, CsF, Li2O, BaO, and the like may be used.

The second electrode 2000 is used as an electron injection electrode and may be formed on the electron injection layer 600 by a method such as a vacuum deposition method or a sputtering method. Various metals may be used to form the second electrode 2000. Specific examples include, but are not limited to, lithium (Li), aluminum (Al), gold (Au), silver (Ag), magnesium (Mg), aluminum-lithium (Al-Li), calcium (Ca), magnesium-indium (Mg-In), and magnesium-silver (Mg-Ag). A transmissive electrode made of ITO or IZO may be used to manufacture front-emission light-emitting devices.

The organic light-emitting device according to the present disclosure may be an organic light-emitting device including the first electrode 1000, the hole injection layer 200, the hole transport layer 300, the light-emitting layer 400, the electron transport layer 500, the electron injection layer 600, the second electrode 2000, and the capping layer 300. The organic light-emitting device may further include one or more intermediate layers if necessary.

On the other hand, the thickness of each organic layer formed according to the present disclosure can be adjusted as desired. Specifically, the thickness may fall within a range of from 1 to 1,000 nm and more specifically a range of from 1 to 150 nm.

The capping layer 3000 may be formed on the outer surface of the first electrode 1000, of the both surfaces of the first electrode 1000. That is, the capping layer 3000 may be formed on the surface that is not adjacent to the hole injection layer 200. In addition, of both surfaces (inner and outer surfaces) of the second electrode 2000, the capping layer 3000 may also be formed on the outer surface of the second electrode 2000. The outer surface is a surface that is not adjacent to the electron injection layer 600. The capping layer 3000 may be formed by a deposition process, and the capping layer 3000 may have a thickness in a range of 100 Å to 3,000 Å and more specifically a range of 300 Å to 2,000 Å. The thickness is adjusted to prevent a decrease in the transmittance of the capping layer 3000.

In addition, although not illustrated in FIG. 1, according to one embodiment of the present disclosure, organic layer having various functions may be additionally formed between the capping layer 3000 and the first electrode 1000 or between the capping layer 3000 and the second electrode 2000. Alternatively, organic layers having various function may be additionally formed on the top surface (outer surface) of the capping layer 3000, and an additional functional layer may be provided in the middle of the capping layer 3000. However, the present disclosure is not limited to the structure described above.

Hereinafter, the present disclosure will be described in greater detail with reference to compound synthesis examples of and examples of organic light-emitting devices (OLEDs).

The OLED examples and compound synthesis examples described below are presented only for illustrative purposes and the scope of the present disclosure is not limited thereby.

### Synthesis Example 1: Synthesis of Compound 97

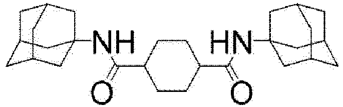

In a round bottom flask, 5.0 g of 1-adamantanamine hydrochloride and 27.8 g of triethylamine were dissolved in 100 ml of 1,4-dioxane. Next, 3.4ml of cyclohexane-1,4-dicarbonyl dichloride dissolved in 35 ml of 1,4-dioxane was slowly added to the flask, stirred for 5 hours at 60°C, and then stirred for 24 hours at room temperature. The reaction solution was added dropwise to 400 ml of a dilute hydrochloric acid solution, and the reaction ended. The precipitated solid was collected by filtering under reduced pressure, and then the solid was recrystallized to produce 6.0 g of Compound 97 (83% yield).
m/z: 438.3246 (100.0%), 439.3280 (30.3%), 440.3313 (4.4%)

### Synthesis Example 2: Synthesis of Compound 103

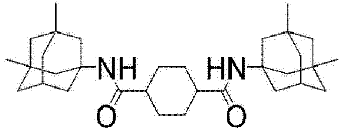

Compound 103 was synthesized using memantine instead of 1-adamantanamine hydrochloride in the same manner as in Synthesis Example 1 (yield 84%).
m/z: 494.3872 (100.0%), 495.3906 (34.6%), 496.3939 (5.8%)

### Synthesis Example 3: Synthesis of Compound 295

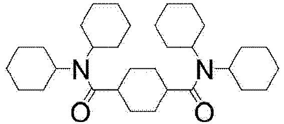

Compound 295 was synthesized using dicyclohexylamine instead of 1-adamantanamine hydrochloride in the same manner as in Synthesis Example 1 (yield 80%).
m/z: 498.4185 (100.0%), 499.4219 (34.6%), 500.4252 (5.8%)

### Synthesis Example 4: Synthesis of Compound 440

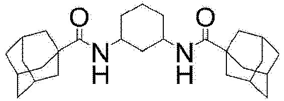

Compound 440 was synthesized using adamantane-1-carbonyl chloride and cyclohexane-1,3-diamine instead of 1-adamantanamine hydrochloride and cyclohexane-1,4-dicarbonyl dichloride in the same manner as Example 1 (yield 83%).
m/z: 438.3246 (100.0%), 439.3280 (30.3%), 440.3313 (4.4%)

### Synthesis Example 5: Synthesis of Compound 441

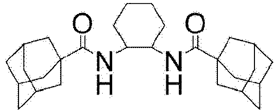

Compound 441 was synthesized using adamantane-1-carbonyl chloride and cyclohexane-1,2-diamine instead of 1-adamantanamine hydrochloride and cyclohexane-1,4-dicarbonyl dichloride in the same manner as Example 1 (yield 87%).
m/z: 438.3246 (100.0%), 439.3280 (30.3%), 440.3313 (4.4%)

### Synthesis Example 6: Synthesis of Compound 566

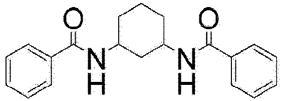

Compound 566 was synthesized using benzoyl chloride and cyclohexane-1,3-diamine instead of 1-adamantanamine hydrochloride and cyclohexane-1,4-dicarbonyl dichloride in the same manner as Example 1 (yield 76%).
m/z: 322.1681 (100.0%), 323.1715 (21.6%), 324.1748 (2.2%)

### Synthesis Examples 6 through 25

Compounds were synthesized using Starting Material 1 and Starting Material 2 shown in Table 1 to Table 3 below instead of 1-adamantanamine hydrochloride and cyclohexane-1,4-dicarbonyl dichloride in the same manner as in Example 1.

**[Table 1]**

| | | Starting Material 1 | Starting Material 2 | m/z |
|---|---|---|---|---|
| Synthesis Example 7 | Compou nd 1 | 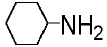 | 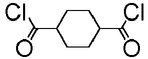 | m/z: 334.26 (100.0%) |
| Synthesis Example 8 | Compou nd 19 | 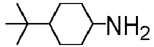 | 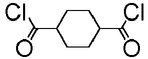 | m/z: 446.39 (100.0%) |

**[Table 2]**

| | | | | |
|---|---|---|---|---|
| Synthesis Example 9 | Compou nd 28 | 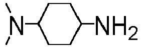 | 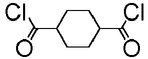 | m/z: 420.35 (100.0%) |
| Synthesis Example 10 | Compou nd 34 | 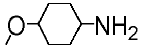 | 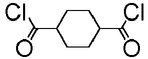 | m/z: 394.28 (100.0%) |
| Synthesis Example 11 | Compou nd 40 | 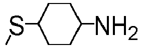 | 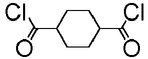 | m/z: 426.24 (100.0%) |
| Synthesis Example 12 | Compou nd 43 | 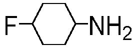 | 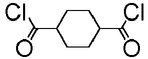 | m/z: 370.24 (100.0%) |
| Synthesis Example 13 | Compou nd 49 | 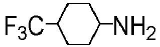 | 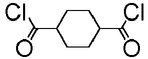 | m/z: 470.24 (100.0%) |
| Synthesis Example 14 | Compou nd 55 | 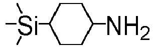 | 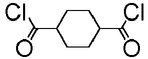 | m/z: 478.34 (100.0%) |
| Synthesis Example 15 | Compou nd 70 | 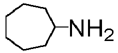 | 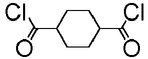 | m/z: 362.29 (100.0%) |
| Synthesis Example 16 | Compou nd 88 | 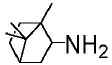 | 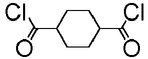 | m/z: 442.36 (100.0%) |
| Synthesis Example 17 | Compou nd 94 | 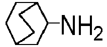 | 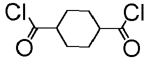 | m/z: 386.29 (100.0%) |
| Synthesis Example 18 | Compou nd 214 | 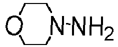 | 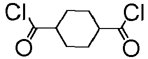 | m/z: 340.21 (100.0%) |
| Synthesis Example 19 | Compou nd 217 | 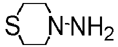 | 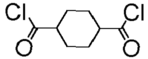 | m/z: 372.17 (100.0%) |
| Synthesis Example 20 | Compou nd 226 | 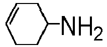 | 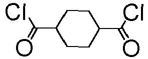 | m/z: 330.23 (100.0%) |
| Synthesis Example 21 | Compou nd 489 | 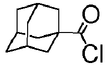 | 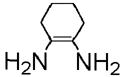 | m/z: 436.31 (100.0%) |
| Synthesis Example 22 | Compou nd 492 | 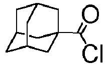 | 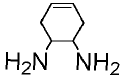 | m/z: 436.31 (100.0%) |

**[Table 3]**

| | | | | |
|---|---|---|---|---|
| Synthesis Example 23 | Compou nd 520 | 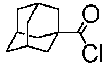 | 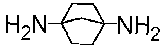 | m/z: 450.32 (100.0%) |
| Synthesis Example 24 | Compou nd 526 | 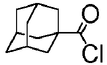 | 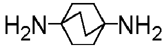 | m/z: 464.34 (100.0%) |
| Synthesis Example 25 | Compou nd 531 | 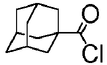 | 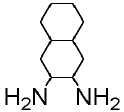 | m/z: 492.37 (100.0%) |

### Manufacturing organic light-emitting device

FIG. 1 illustrates the construction of a typical organic light-emitting device. In the present disclosure, an organic light-emitting device having the structure shown in FIG. 1 was manufactured as an example. Specifically, in the manufactured organic light-emitting device includes a, an anode (hole injection electrode 1000), a hole injection layer 200, a hole transport layer 300, a light-emitting layer 400, an electron transport layer 500, an electron injection layer 600, a cathode (electron injection electrode 2000), and a capping layer 3000 are stacked in this order from the bottom. The capping layer 3000 may be a multilayer structure in which the first capping layer and the second capping layer are combined.

When manufacturing the organic light-emitting device, as a substrate 10, a transparent glass substrate or a flexible plastic substrate is used.

In the organic light-emitting device, the hole injection electrode 1000 is used as the anode. For the hole injection electrode, a material having a low work function is used to enable hole injection. Specifically, the hole injection electrode is made of a transparent material such as indium tin oxide (ITO), indium zinc oxide (IZO), or graphene.

The materials used for the hole injection layer 200, hole transport layer 300, light-emitting layer 400, electron transport layer 500, electron injection layer 600, and high refractive index capping layer are summarized in Table 4 below.

In addition, the cathode 2000 for electron injection was formed on the electron injection layer 600. Various metals can be used to form the cathode. Specific examples of the metal include aluminum, gold, silver, magnesium, and magnesium-silver alloys.

**[Table 4]**

| | | |
|---|---|---|
| 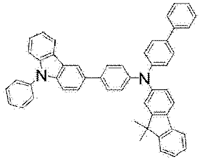 | 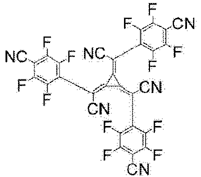 | 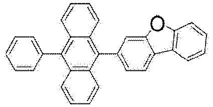 |
| HT01 | NDP9 | BH01 |
| 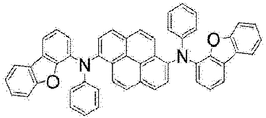 | 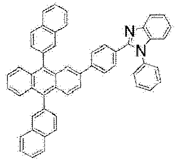 | 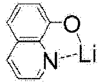 |
| BD01 | ET01 | Liq |
| 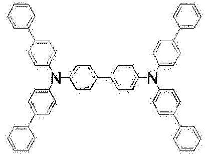 | | |
| CPM01 (n 2.13@450nm) | | |

### Example 1

An indium tin oxide (ITO) substrate with a reflective layer containing silver (Ag) was cleaned with distilled water by ultrasonic cleaning. After the completion of the distilled water washing, the substrate was cleaned with a solvent such as isopropyl alcohol, acetone, or methanol by ultrasonic cleaning and then dried. As a hole-injection layer, HT01 doped with 3% by weight of NDP9 was formed to have a thickness of 100 Å on top of the ITO substrate. Next, HT01 with a thickness of 1000 Å was deposited thereon as a hole transport layer. BH01 (host layer) doped with 3% by weight of BD01 (dopant) was formed to have a thickness of 250 Å as a light-emitting layer. Next, an electron transport layer with a thickness of 300 Å was deposited using a mixture of ET01 and Liq (in a weight ratio of 1:1), and then LiF was deposited to form an electron injection layer with a thickness of 10 Å. MgAg was then deposited to a thickness of 15 nm to form a cathode. Next, a high refractive index capping layer with a thickness of 950 Å was formed on the cathode by depositing CPM01, and a low refractive index capping layer with a thickness of 400 Å was then formed by depositing the compound prepared in Synthesis Example 1. The resulting structure was encapsulated in a glove box to produce an organic light-emitting device.

### Examples 2 to 25

Organic light-emitting devices were manufactured in the same manner as in Example 1 except that the compounds prepared in Synthesis Examples 2 to 25 were used, respectively, to form the respective low refractive index capping layers.

### Comparative Examples 1 to 4

Organic light-emitting devices were manufactured in the same manner as in Example 1 except that Comparative Compounds 1 to 5 listed in Table 5 were respectively used to form the respective low refractive index capping layers.

**[Table 5]**

| | | |
|---|---|---|
| 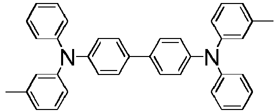 | 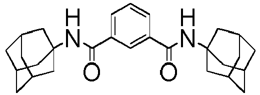 | 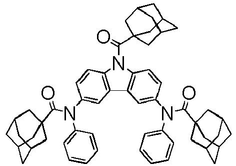 |
| Comparative Comound 1 | Comparative Comound 2 | Comparative Comound 3 |
| 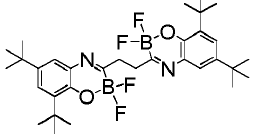 | | |
| Comparative Comound 4 | | |

### <Experimental Example 1> Performance evaluation of organic light-emitting devices

Electrons and holes were injected by applying a voltage using a source measure unit (Kiethley 2400 manufactured by Keithley Instruments, Inc.) and the luminance was measured using a spectroradiometer (CS-2000 manufactured by Konica Minolta Inc.) when light is emitted. To evaluate the performance of each of the organic light-emitting devices of Examples 1 to 6, 16 to 18, 21 to 24, and Comparative Examples 1 to 4, the current density and luminance with respect to the applied voltage were measured under atmospheric pressure, and the results are shown in Table 6.

**[Table 6]**

| | Op. V | mA/cm2 | Cd/A | CIEx | ClEy | LT97 |
|---|---|---|---|---|---|---|
| Example 1 | 3.45 | 10 | 10.11 | 0.140 | 0.043 | 170 |
| Example 2 | 3.45 | 10 | 10.15 | 0.140 | 0.043 | 185 |
| Example 3 | 3.45 | 10 | 12.07 | 0.139 | 0.042 | 161 |
| Example 4 | 3.44 | 10 | 11.13 | 0.139 | 0.042 | 178 |
| Example 5 | 3.45 | 10 | 11.70 | 0.139 | 0.042 | 182 |
| Example 6 | 3.45 | 10 | 9.41 | 0.139 | 0.042 | 153 |
| Example 16 | 3.45 | 10 | 11.90 | 0.139 | 0.042 | 166 |
| Example 17 | 3.45 | 10 | 11.85 | 0.139 | 0.042 | 166 |
| Example 18 | 3.45 | 10 | 11.93 | 0.139 | 0.042 | 158 |
| Example 21 | 3.45 | 10 | 10.85 | 0.139 | 0.042 | 172 |
| Example 22 | 3.45 | 10 | 10.85 | 0.139 | 0.042 | 172 |
| Example 23 | 3.45 | 10 | 11.00 | 0.139 | 0.042 | 174 |
| Example 24 | 3.45 | 10 | 11.05 | 0.139 | 0.042 | 175 |
| Comparative Example 1 | 3.47 | 10 | 6.46 | 0.134 | 0.054 | 101 |
| Comparative Example 2 | 3.46 | 10 | 8.22 | 0.135 | 0.047 | 144 |
| Comparative Example 3 | 3.46 | 10 | 7.44 | 0.137 | 0.050 | 123 |
| Comparative Example 4 | 3.46 | 10 | 8.30 | 0.137 | 0.047 | 75 |

Comparing the examples of the present invention with the comparative examples, the present invention has a cycloalkylene core, so it has a low refractive index by forming a low polarizability within the molecule, and at the same time, it has an even lower refractive index because two or more aminebased substituents are connected. At the same time, thin film formation and thermal stability are excellent, making it possible to provide organic light-emitting devices with high color purity, high efficiency, and long lifespan.

Specifically, comparing Example 1 and Example 2, by having an additional substituent, thermal stability is excellent, and the lifespan can be effectively improved. Comparing Example 1 and Example 3, the structure with an adamantane group as a substituent has excellent thermal stability and can effectively improve lifespan, and the structure with cyclohexylene has a lower refractive index, and can effectively improve efficiency. Additionally, comparing Example 1 with Examples 4 and 5, the structure in which the amine is directly bonded to the linking group can have better thermal stability and a lower refractive index. In particular, when the cycloalkylene core is 1,2-cycloalkylene, it has a lower refractive index and is effective in improving efficiency and lifespan. Lastly, comparing Examples 1 to 5 with Example 6, it can be seen that when a cycloalkyl group with a low polarizability is used as a substituent, a low refractive index is maintained and efficiency is effectively improved.

### <Experimental Example 2> Evaluation of refractive index

With the use of each of the compounds of Synthesis Examples 1 to 6 and Comparative Compounds 1 to 4, deposition films with a thickness of 30 nm were formed on respective silicon substrates, and the refractive index at a wavelength of 450 nm was measured using an ellipsometer device (M-2000X available from J.A.Woollam Co., Inc.). The measurement results are summarized in Table 7.

**[Table 7]**

| | Compara tive Compound 1 | Compara tive Compound 2 | Compara tive Compound 3 | Compara tive Compound 4 | Compo und 97 | Compo und 103 | Compo und 295 | Compou nd 440 | Compou nd 441 | Compo und 566 |
|---|---|---|---|---|---|---|---|---|---|---|
| n@450nm | 1.88 | 1.59 | 1.74 | 1.58 | 1.49 | 1.49 | 1.44 | 1.47 | 1.45 | 1.54 |

Referring to Table 7 above, it can be seen that the compounds according to the present disclosure exhibit a low refractive index of 1.55 or less at a wavelength of 450 nm. In addition, other compounds (not listed in Table 7) according to the present disclosure also exhibited a low refractive index of 1.55 or less at a wavelength of 450 nm.

## Claims

1. A compound for a capping layer, the compound being represented by Formula 1 below: 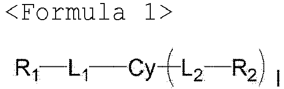 (in Formula 1,
Cy is a substituted or unsubstituted C3∼C50 cycloalkylene group, or a C3~C50 cycloalkenylene group,
L and L1 are each independently a direct bond, a substituted or unsubstituted C1~C50 alkylene group, a substituted or unsubstituted C2~C50 alkenylene group, a substituted or unsubstituted C1~C50 alkyleneoxy group, an ether group, a substituted or unsubstituted C1~C50 sulfide group, a thioether group, a substituted or unsubstituted C1~C50 carbonyl group, substituted or unsubstituted -C(X1)NR3-, substituted or unsubstituted -NR4C(X2)-, substituted or unsubstituted -NR-, a substituted or unsubstituted C3∼C50 cycloalkylene group, a substituted or unsubstituted C1~C50 heterocyclylene group, or a combination thereof,
X1 and X2 are each independently O, S, Se, Te, NR5, or CR6R7,
R and R1 to R7 are each independently hydrogen, deuterium, halogen, a nitro group, a nitrile group, a hydroxy group, a thiol group, a substituted or unsubstituted amino group, a substituted or unsubstituted C1 to C50 alkyl group, a substituted or unsubstituted C2~C50 alkenyl group, a substituted or unsubstituted C1~C50 alkoxy group, a substituted or unsubstituted C1~C50 sulfide group, a substituted or unsubstituted silyl group, a substituted or unsubstituted C3~C50 cycloalkyl group, a substituted or unsubstituted C1~C50 heterocyclyl group, a substituted or unsubstituted C3∼C50 aryl group, or a substituted or unsubstituted C2∼C50 heteroaryl group, and a plurality of adjacent R, R1 to R7 may or may not form a ring by bonding with each other,
l is an integer in a range of from 1 to 5).

2. The compound for a capping layer of claim 1, wherein Formula 1 is represented by Formula 2 or Formula 3 below: 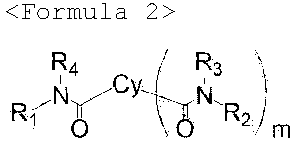 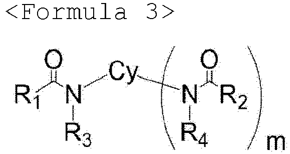 (in Formulas 2 to 3,
the same symbols as in Formula 1 above have the same definitions as in Formula 1,
m is an integer in a range of from 1 to 5).

3. The compound for a capping layer of claim 1, wherein in Formula 1, one or more of R1 and R2 are a substituted or unsubstituted C3∼C50 cycloalkyl group, or a substituted or unsubstituted C1~C50 heterocyclyl group.

4. The compound for a capping layer of claim 3, wherein substituents of R1 and R2 are each independently selected from the group consisting of hydroxyl groups, thiol groups, amino groups, C1-C30 alkyl groups, C1-C30 alkoxy groups, C1-C30 sulfide groups, silyl groups, halogen groups, C3-C30 cycloalkyl groups, C1-C30 heterocyclyl groups, and combinations thereof.

5. The compound for a capping layer of claim 1, wherein Cy is selected from Structural Formula Formulas A-1 to A-15: 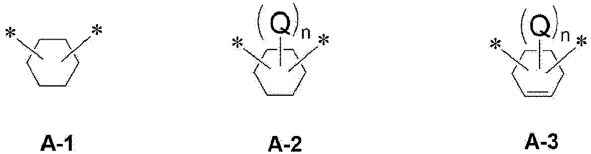 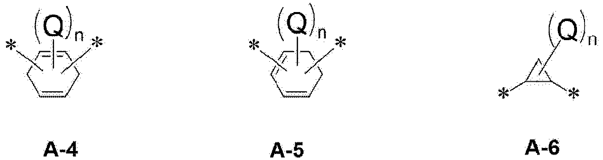 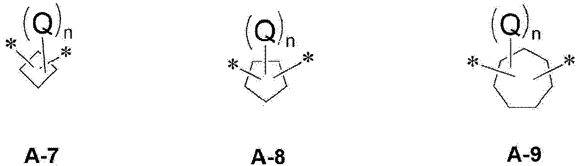 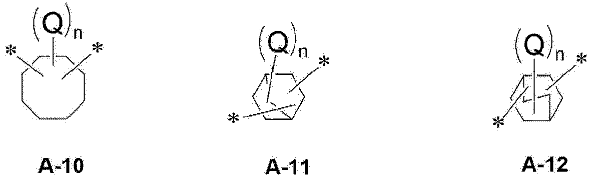 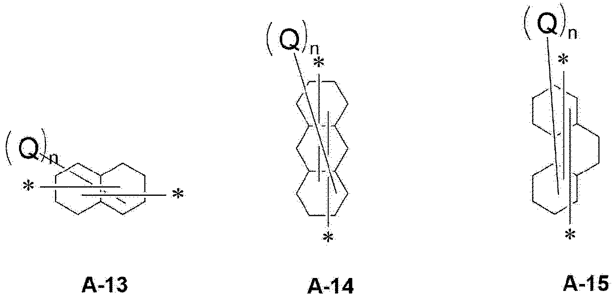 (in Structural Formulas A-1 to A-15, Q is each independently a methyl group, an ethyl group, a t-butyl group, a cyclohexyl group, an adamantane group, a dihydroamine group, a dimethylamine group, a hydroxy group, a methoxy group, a mercaptan group, methylthio group, a fluoro group, a trifluoromethyl group, or a trimethylsilyl group,
n is each independently an integer in a range of from 0 to 8,
* indicates a bonding site).

6. The compound for a capping layer of claim 1, wherein when l is 1, Cy is a cycloalkylene where -L1-R1 and -L2-R2 are bonded to the two immediately adjacent carbons or a cycloalkylene in which -L1-R1 and -L2-R2 are each bonded to two carbons immediately adjacent to either side of any carbon.

7. The compound for a capping layer of claim 1, wherein Cy is 1,2-cyclopentylene, 1,2-cyclohexylene, 1,2-cycloheptylene, 1,2-cyclooctylene, 1,2-cyclononylene, 1-2-cyclodecylene , 1,3-cyclopentylene, 1,3-cyclohexylene, 1,3-cycloheptylene, 1,3-cyclooctylene, 1,3-cyclononylene, 1,3-cyclodecylene, 1 , 2-cyclohex-1-ene, 4,5-cyclohex-1-ene, or 2,3-decahydronaphthalene.

8. The compound for a capping layer of claim 1, wherein in Formula 1, at least one of -L1-R1 and -L2-R2 is independently selected from Structural Formulas B-1 to B-47: 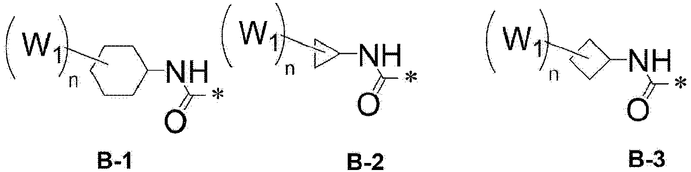 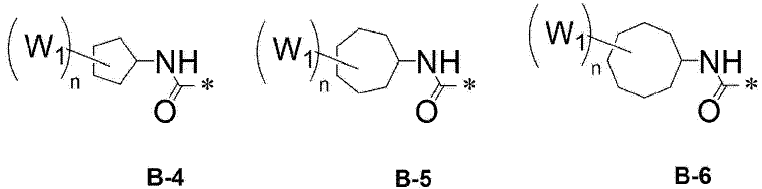 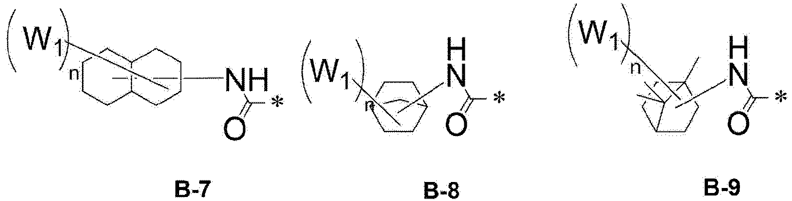 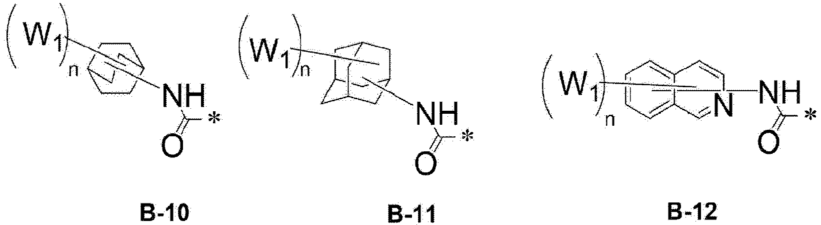 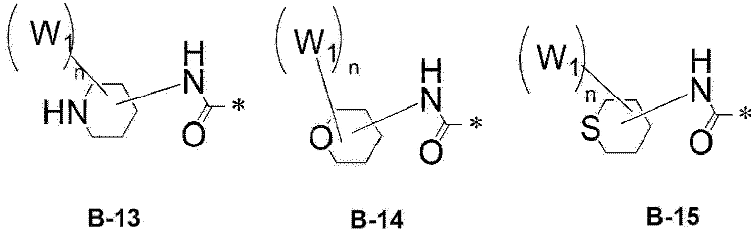 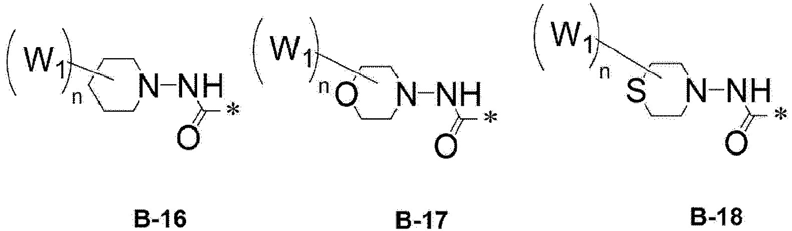 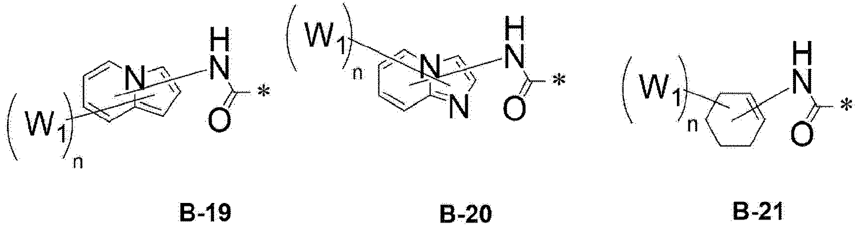 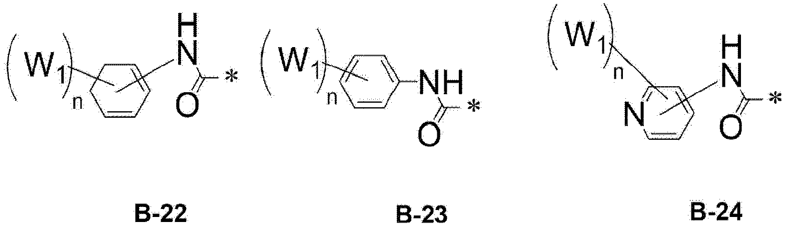 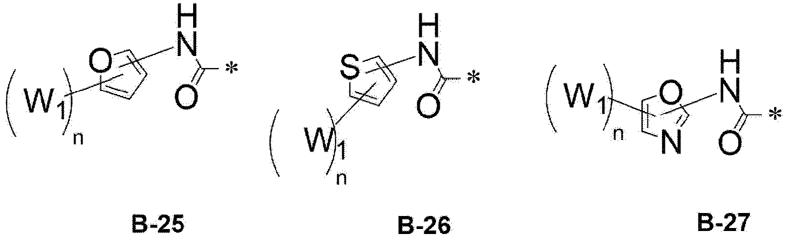 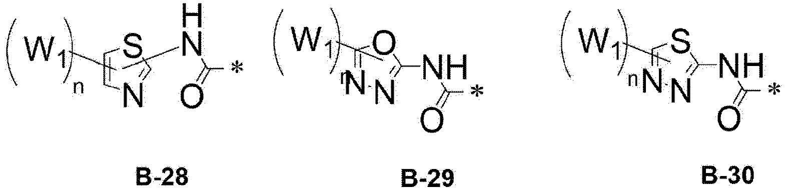 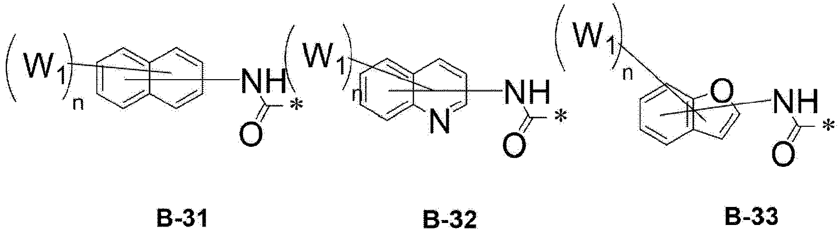 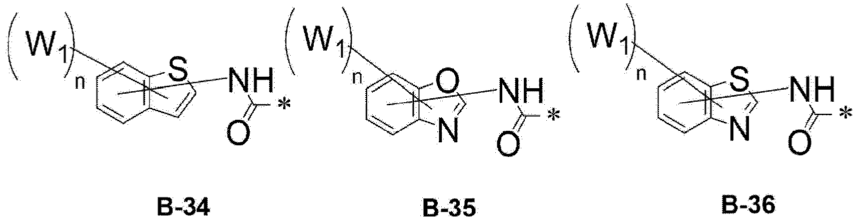 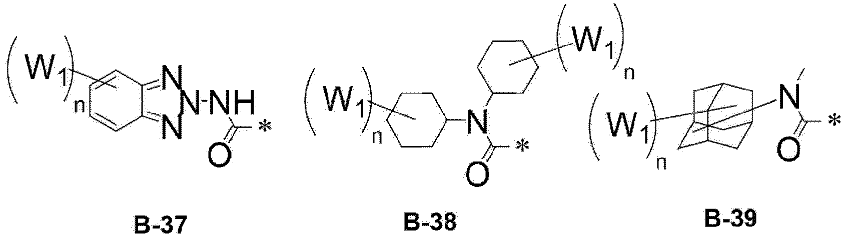 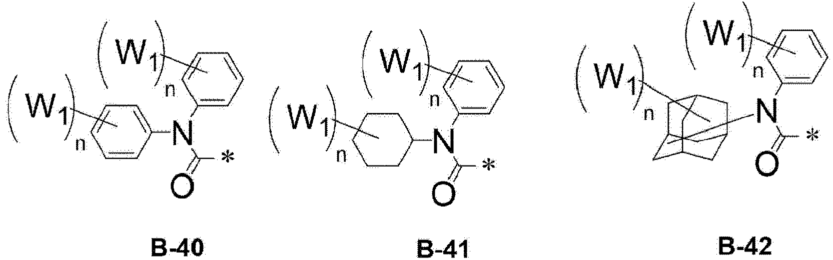 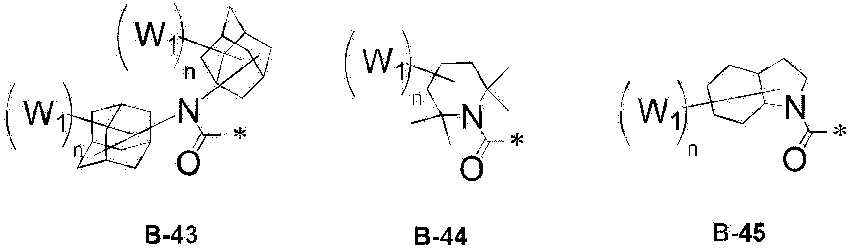 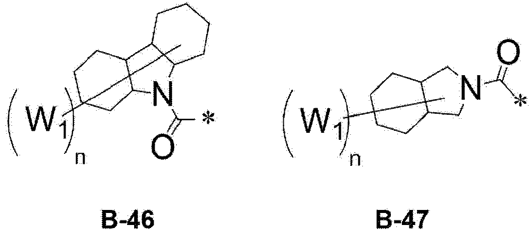 (in the structural Formulas, W1 is each independently a methyl group, an ethyl group, a t-butyl group, a cyclohexyl group, an adamantane group, a dihydroamine group, a dimethylamine group, a hydroxy group, a methoxy group, a mercaptan group, methylthio group, a fluoro group, a trifluoromethyl group, or a trimethylsilyl group,
n is each independently an integer in a range of from 0 to 10,
* indicates a bonding site).

9. The compound for a capping layer of claim 8, wherein at least one of -L1-R1 and -L2-R2 has each independently a structural formula in which -NH-CO-* in Structural Formulas B-1 to B-37 is replaced by -Z-* or *-Z-, in which Z is -NH-CS-, -NH-C(=NH)-, -CH2-NH-CO-, -CH2-CO-NH-, -O-NH-CO-, -S-NH-CO-, - CO-NH-CO-, -NH-C(=NMe)-, -NHC(=CHMe)-, -NHCOO-, -NH-, -CO-NH-, -OCONH-, -SCONH-, -CO-CO-NH-, -CH2-, -O-, -S-, -C0-, or "-".

10. The compound for a capping layer of claim 8, wherein at least one of -L1-R1 and -L2-R2 has each independently a structural formula in which N-C0-* structure in Structural Formulas B-38 to B-47 is replaced by N-CS-*, N-C(=NH)-*, N-C(=NMe)-*, NC(=CHMe)-*, NCOO-*, N-*, NCH2-*, NO-*, or NS-*.

11. The compound for a capping layer of claim 1, wherein in Formula 1, at least one of -L1-R1 and -L2-R2 is independently selected from Structural Formulas C-1 through C-12:

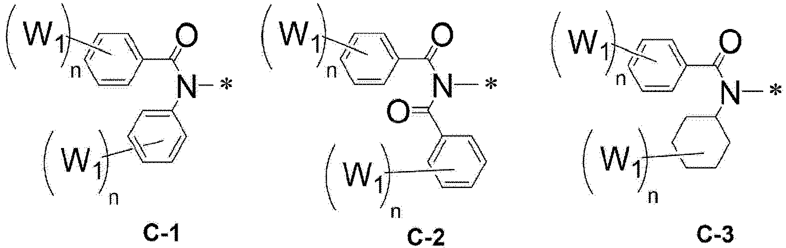

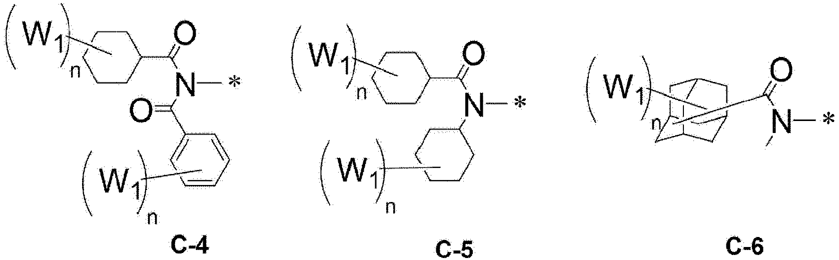

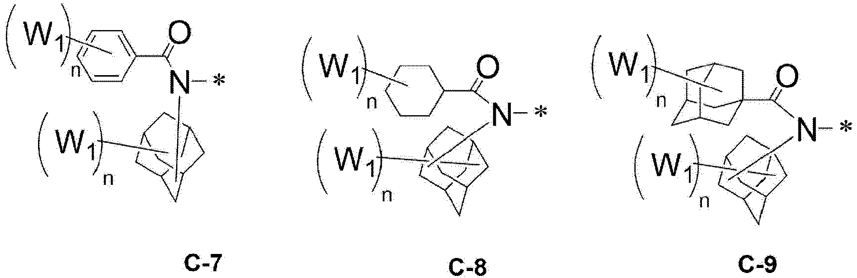

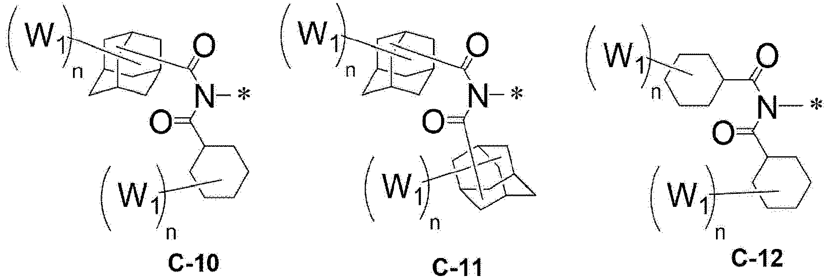

12. The compound for a capping layer of claim 11, wherein at least one of -L1-R1 and -L2-R2 has independently a structural formula in which -CO-N-* structure in Structural Formulas C-1 through C-12 is replaced by -N-*.

13. The compound for a capping layer of claim 1, wherein the compound is any one of the following compounds:
| | | |
|---|---|---|
| 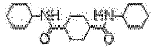 | 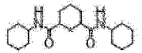 | 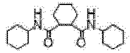 |
| **1** | **2** | **3** |
| 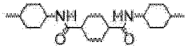 | 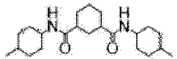 | 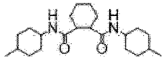 |
| **4** | **5** | **6** |
| 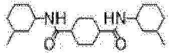 | 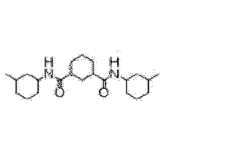 | 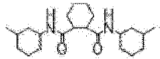 |
| **7** | **8** | **9** |
| 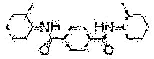 | 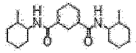 | 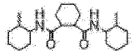 |
| **10** | **11** | **12** |
| 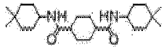 | 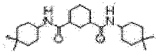 | 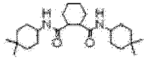 |
| **13** | **14** | **15** |
| 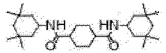 | 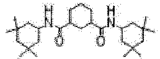 | 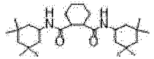 |
| **16** | **17** | **18** |
| 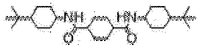 | 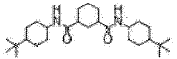 | 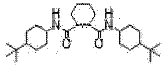 |
| **19** | **20** | **21** |
| 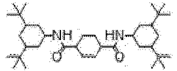 | 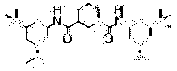 | 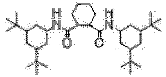 |
| **22** | **23** | **24** |
| 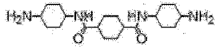 | 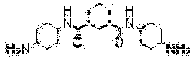 | 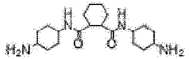 |
| **25** | **26** | **27** |
| 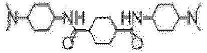 | 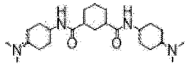 | 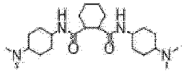 |
| **28** | **29** | **30** |
| 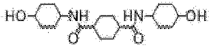 | 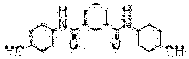 | 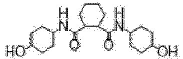 |
| **31** | **32** | **33** |
| 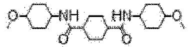 | 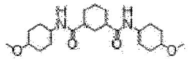 | 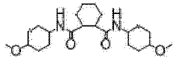 |
| **34** | **35** | **36** |
| 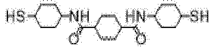 | 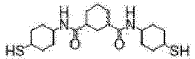 | 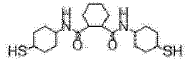 |
| **37** | **38** | **39** |
| 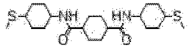 | 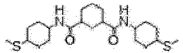 | 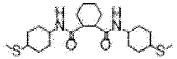 |
| **40** | **41** | **42** |
| 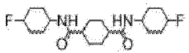 | 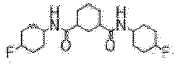 | 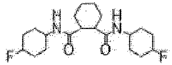 |
| **43** | **44** | **45** |
| 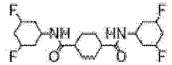 | 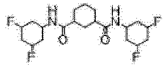 | 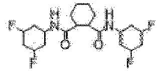 |
| **46** | **47** | **48** |
| 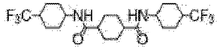 | 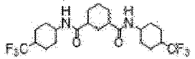 | 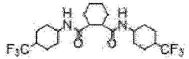 |
| **49** | **50** | **51** |
| 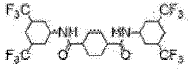 | 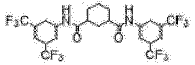 | 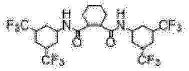 |
| **52** | **33** | **54** |
| 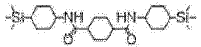 | 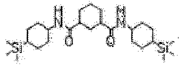 | 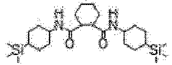 |
| **55** | **56** | **57** |
| 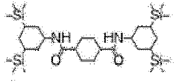 | 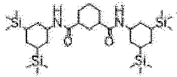 | 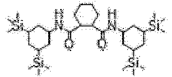 |
| **58** | **59** | **60** |
| 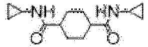 | 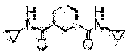 | 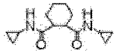 |
| **61** | **62** | **63** |
| 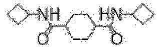 | 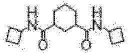 | 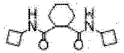 |
| **64** | **65** | **66** |
| 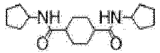 | 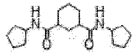 | 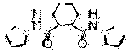 |
| **67** | **68** | **69** |
| 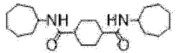 | 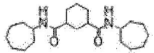 | 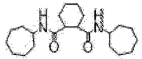 |
| **70** | **71** | **72** |
| 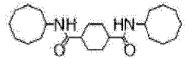 | 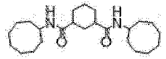 | 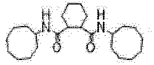 |
| **73** | **74** | **75** |
| 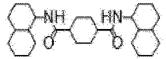 | 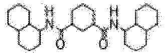 | 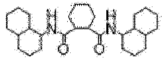 |
| **76** | **77** | **78** |
| 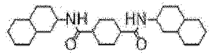 | 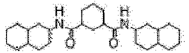 | 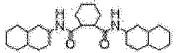 |
| **79** | **80** | **81** |
| 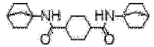 | 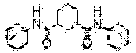 | 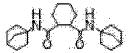 |
| **82** | **83** | **84** |
| 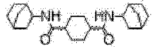 | 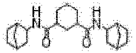 | 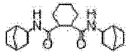 |
| **85** | **86** | **87** |
| 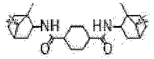 | 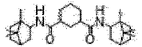 | 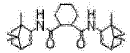 |
| **88** | **89** | **90** |
| 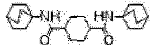 | 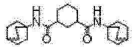 | 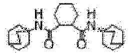 |
| **91** | **92** | **93** |
| 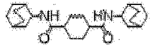 | 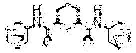 | 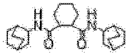 |
| **94** | **95** | **96** |
| 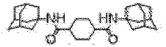 | 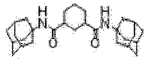 | 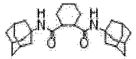 |
| **97** | **98** | **99** |
| 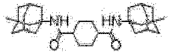 | 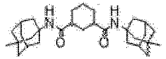 | 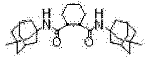 |
| **100** | **101** | **102** |
| 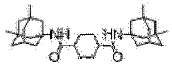 | 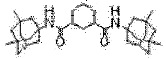 | 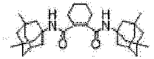 |
| **103** | **104** | **105** |
| 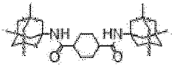 | 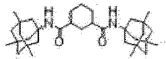 | 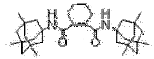 |
| **106** | **107** | **108** |
| 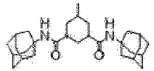 | 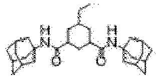 | 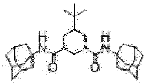 |
| **109** | **110** | **111** |
| 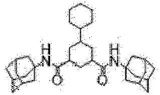 | 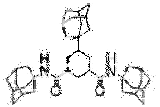 | 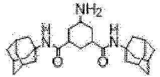 |
| **112** | **113** | **114** |
| 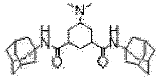 | 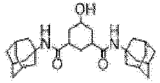 | 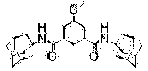 |
| **115** | **116** | **117** |
| 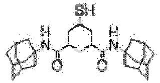 | 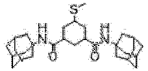 | 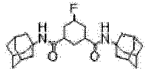 |
| **118** | **119** | **120** |
| 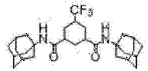 | 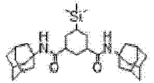 | 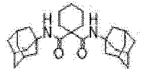 |
| **121** | **122** | **123** |
| 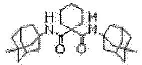 | 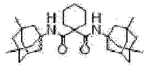 | 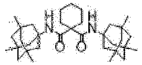 |
| **124** | **125** | **126** |
| 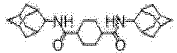 | 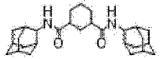 | 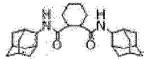 |
| **127** | **128** | **129** |
| 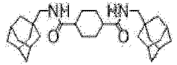 | 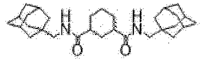 | 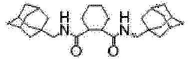 |
| **130** | **131** | **132** |
| 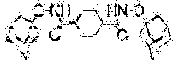 | 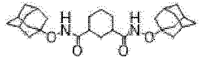 | 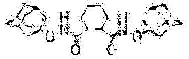 |
| **133** | **134** | **135** |
| 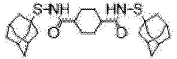 | 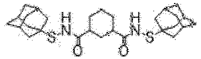 | 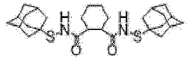 |
| **136** | **137** | **138** |
| 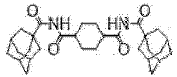 | 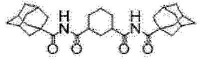 | 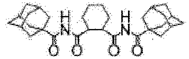 |
| **139** | **140** | **141** |
| 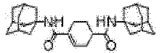 | 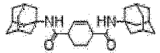 | 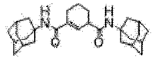 |
| **142** | **143** | **144** |
| 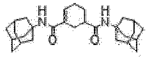 | 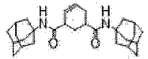 | 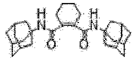 |
| **145** | **146** | **147** |
| 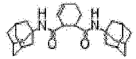 | 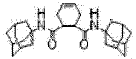 | 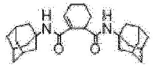 |
| **148** | **149** | **150** |
| 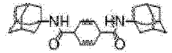 | 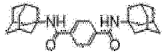 | 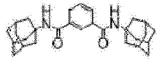 |
| **151** | **152** | **153** |
| 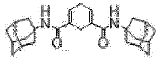 | 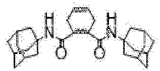 | 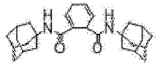 |
| **154** | **155** | **156** |
| 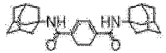 | 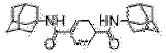 | 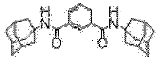 |
| **157** | **158** | **159** |
| 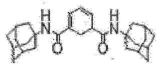 | 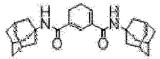 | 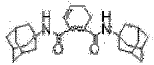 |
| **160** | **161** | **162** |
| 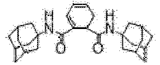 | 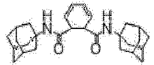 | 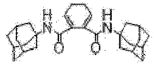 |
| **163** | **164** | **165** |
| 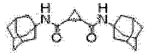 | 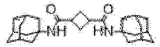 | 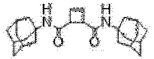 |
| **166** | **167** | **168** |
| 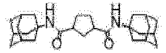 | 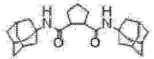 | 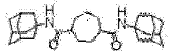 |
| **169** | **170** | **171** |
| 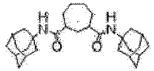 | 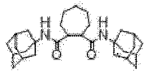 | 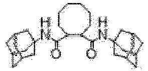 |
| **172** | **173** | **174** |
| 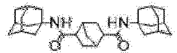 | 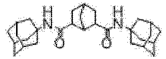 | 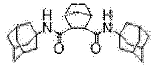 |
| **175** | **176** | **177** |
| 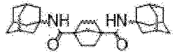 | 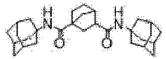 | 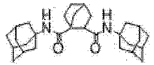 |
| **178** | **179** | **180** |
| 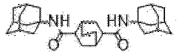 | 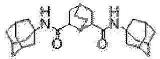 | 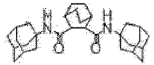 |
| **181** | **182** | **183** |
| 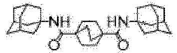 | 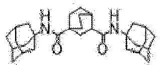 | 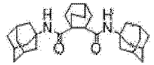 |
| **184** | **185** | **186** |
| 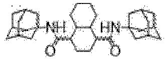 | 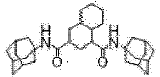 | 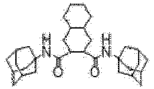 |
| **187** | **188** | **189** |
| 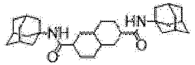 | 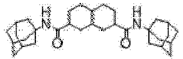 | 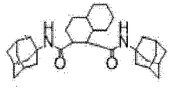 |
| **190** | **191** | **192** |
| 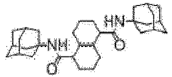 | 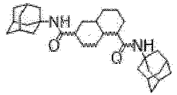 | 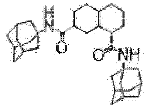 |
| **193** | **194** | **195** |
| 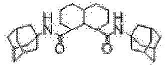 | 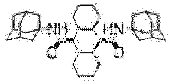 | 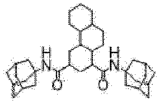 |
| **196** | **197** | **198** |
| 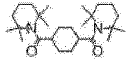 | 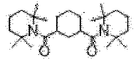 | 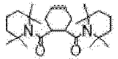 |
| **199** | **200** | **201** |
| 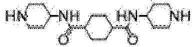 | 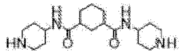 | 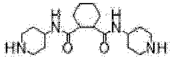 |
| **202** | **203** | **204** |
| 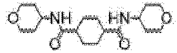 | 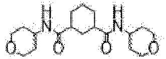 | 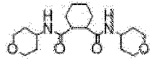 |
| **205** | **206** | **207** |
| 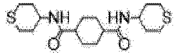 | 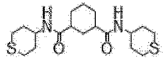 | 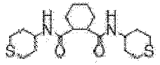 |
| **208** | **209** | **210** |
| 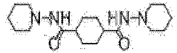 | 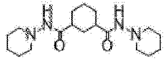 | 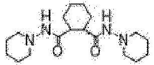 |
| **211** | **212** | **213** |
| 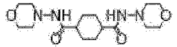 | 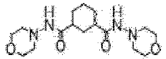 | 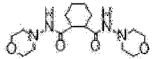 |
| **214** | **215** | **216** |
| 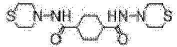 | 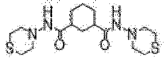 | 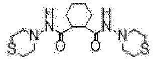 |
| **217** | **218** | **219** |
| 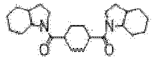 | 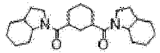 | 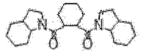 |
| **220** | **221** | **222** |
| 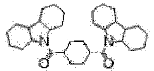 | 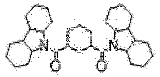 | 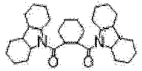 |
| **223** | **224** | **225** |
| 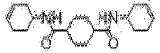 | 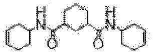 | 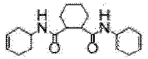 |
| **226** | **227** | **228** |
| 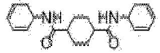 | 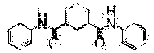 | 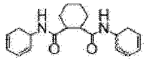 |
| **229** | **230** | **231** |
| 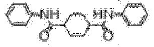 | 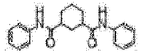 | 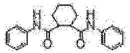 |
| **232** | **233** | **234** |
| 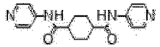 | 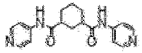 | 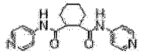 |
| **235** | **236** | **237** |
| 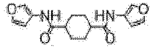 | 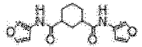 | 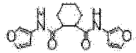 |
| **238** | **239** | **240** |
| 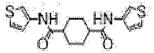 | 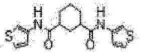 | 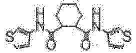 |
| **241** | **242** | **243** |
| 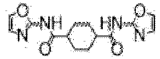 | 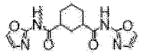 | 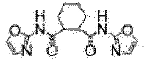 |
| **244** | **245** | **246** |
| 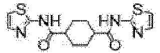 | 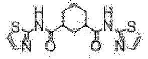 | 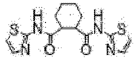 |
| **247** | **248** | **249** |
| 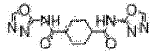 | 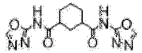 | 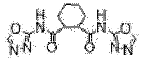 |
| **250** | **251** | **252** |
| 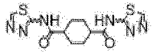 | 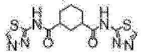 | 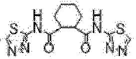 |
| **253** | **254** | **255** |
| 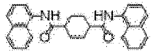 | 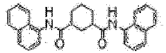 | 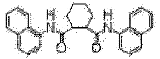 |
| **256** | **257** | **258** |
| 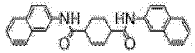 | 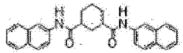 | 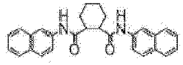 |
| **259** | **260** | **261** |
| 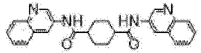 | 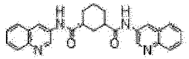 | 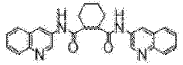 |
| **262** | **263** | **264** |
| 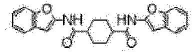 | 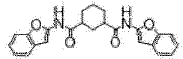 | 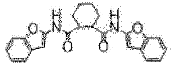 |
| **265** | **266** | **267** |
| 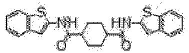 | 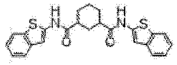 | 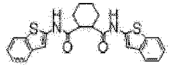 |
| **268** | **269** | **270** |
| 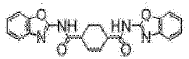 | 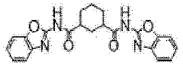 | 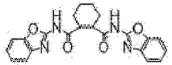 |
| **271** | **212** | **273** |
| 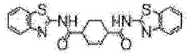 | 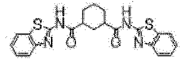 | 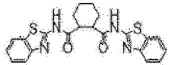 |
| **274** | **275** | **276** |
| 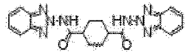 | 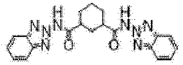 | 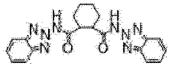 |
| **277** | **218** | **279** |
| 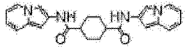 | 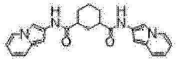 | 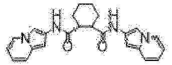 |
| **280** | **281** | **282** |
| 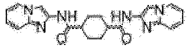 | 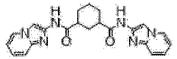 | 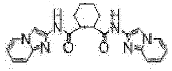 |
| **283** | **284** | **285** |
| 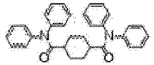 | 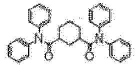 | 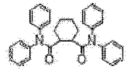 |
| **286** | **287** | **288** |
| 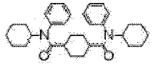 | 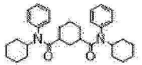 | 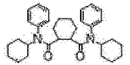 |
| **289** | **290** | **291** |
| 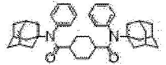 | 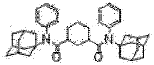 | 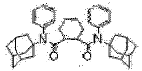 |
| **292** | **293** | **294** |
| 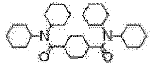 | 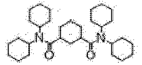 | 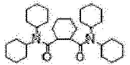 |
| **295** | **296** | **297** |
| 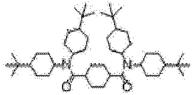 | 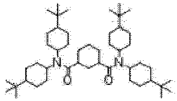 | 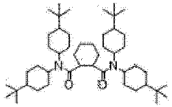 |
| **298** | **299** | **300** |
| 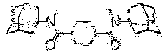 | 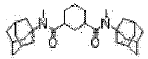 | 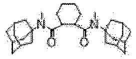 |
| **301** | **302** | **303** |
| 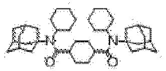 | 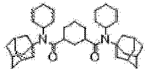 | 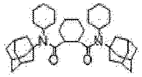 |
| **304** | **305** | **306** |
| 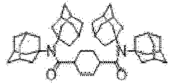 | 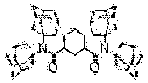 | 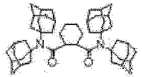 |
| **307** | **308** | **309** |
| 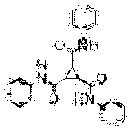 | 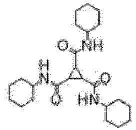 | 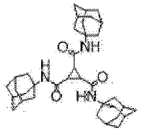 |
| **310** | **311** | **312** |
| 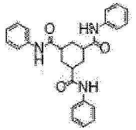 | 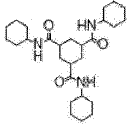 | 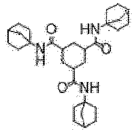 |
| **313** | **314** | **315** |
| 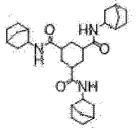 | 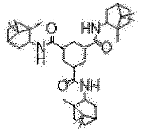 | 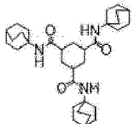 |
| **316** | **317** | **318** |
| 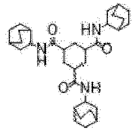 | 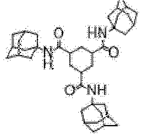 | 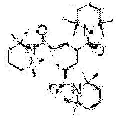 |
| **319** | **320** | **321** |
| 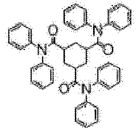 | 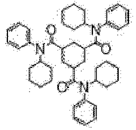 | 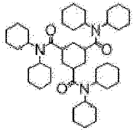 |
| **322** | **323** | **324** |
| 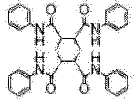 | 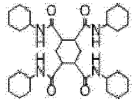 | 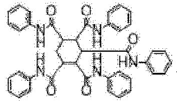 |
| **325** | **326** | **327** |
| 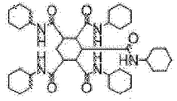 | 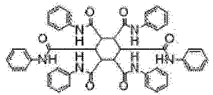 | 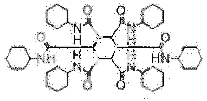 |
| **328** | **329** | **330** |
| 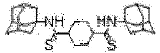 | 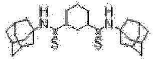 | 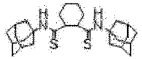 |
| **331** | **332** | **333** |
| 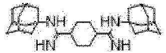 | 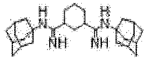 | 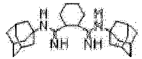 |
| **334** | **335** | **336** |
| 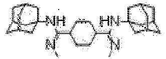 | 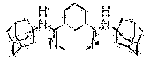 | 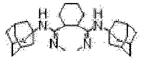 |
| **337** | **338** | **339** |
| 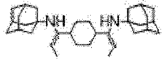 | 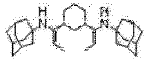 | 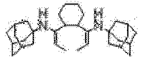 |
| **340** | **341** | **342** |
| 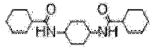 | 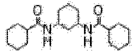 | 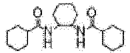 |
| **343** | **344** | **345** |
| 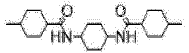 | 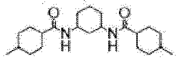 | 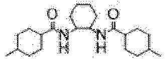 |
| **346** | **347** | **348** |
| 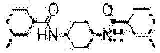 | 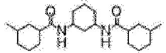 | 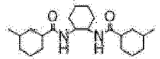 |
| **349** | **350** | **351** |
| 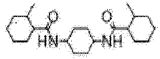 | 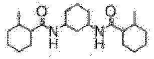 | 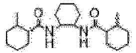 |
| **352** | **353** | **354** |
| 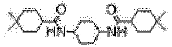 | 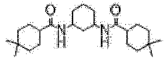 | 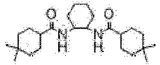 |
| **355** | **356** | **357** |
| 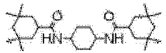 | 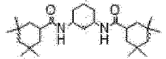 | 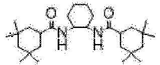 |
| **358** | **359** | **360** |
| 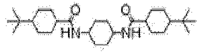 | 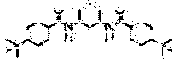 | 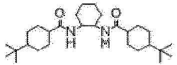 |
| **361** | **362** | **363** |
| 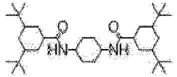 | 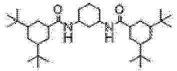 | 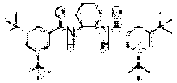 |
| **364** | **365** | **366** |
| 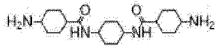 | 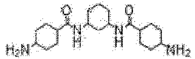 | 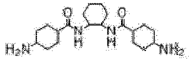 |
| **367** | **368** | **369** |
| 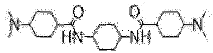 | 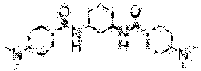 | 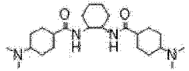 |
| **370** | **371** | **372** |
| 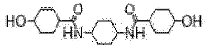 | 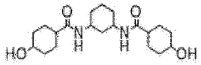 | 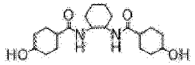 |
| **373** | **374** | **375** |
| 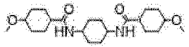 | 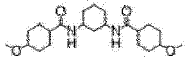 | 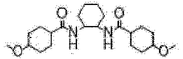 |
| **376** | **377** | **378** |
| 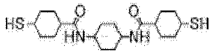 | 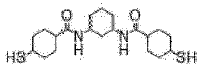 | 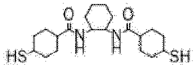 |
| **379** | **380** | **381** |
| 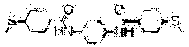 | 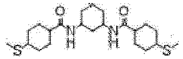 | 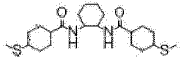 |
| **382** | **383** | **384** |
| 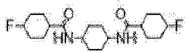 | 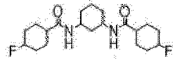 | 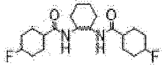 |
| **385** | **386** | **387** |
| 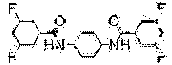 | 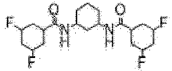 | 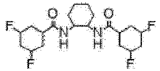 |
| **388** | **389** | **390** |
| 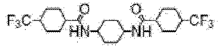 | 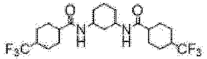 | 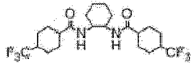 |
| **391** | **392** | **393** |
| 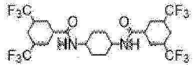 | 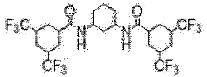 | 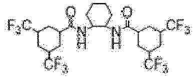 |
| **394** | **395** | **396** |
| 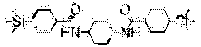 | 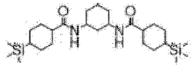 | 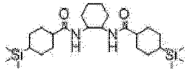 |
| **397** | **398** | **399** |
| 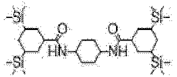 | 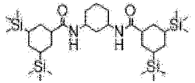 | 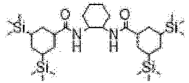 |
| **400** | **401** | **402** |
| 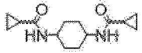 | 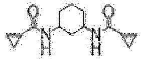 | 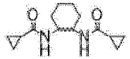 |
| **403** | **404** | **405** |
| 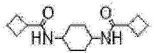 | 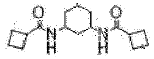 | 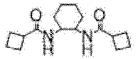 |
| **406** | **407** | **408** |
| 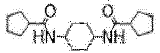 | 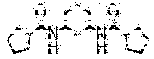 | 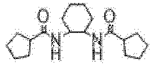 |
| **409** | **410** | **411** |
| 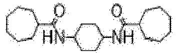 | 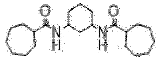 | 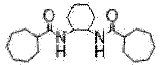 |
| **412** | **413** | **414** |
| 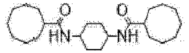 | 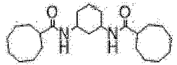 | 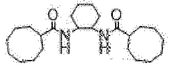 |
| **415** | **416** | **417** |
| 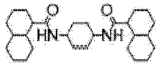 | 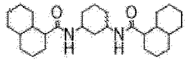 | 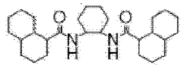 |
| **418** | **419** | **420** |
| 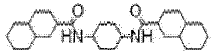 | 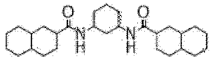 | 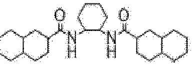 |
| **421** | **422** | **423** |
| 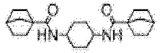 | 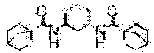 | 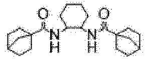 |
| **424** | **425** | **426** |
| 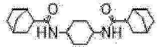 | 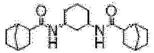 | 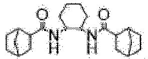 |
| **427** | **428** | **429** |
| 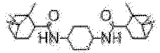 | 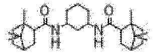 | 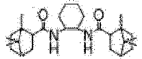 |
| **430** | **431** | **432** |
| 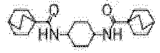 | 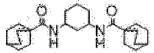 | 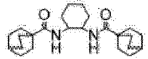 |
| **433** | **434** | **435** |
| 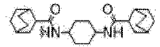 | 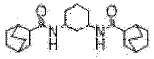 | 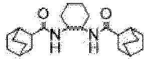 |
| **436** | **437** | **438** |
| 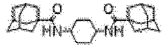 | 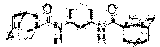 | 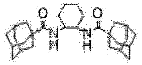 |
| **439** | **440** | **441** |
| 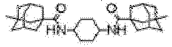 | 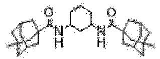 | 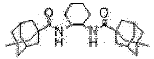 |
| **442** | **443** | **444** |
| 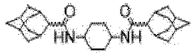 | 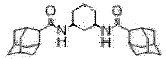 | 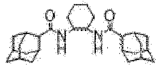 |
| **445** | **446** | **447** |
| 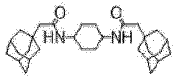 | 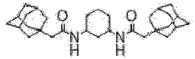 | 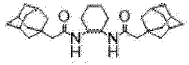 |
| **448** | **449** | **450** |
| 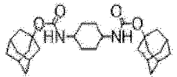 | 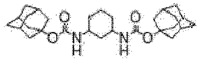 | 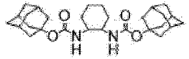 |
| **451** | **452** | **453** |
| 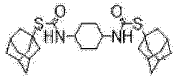 | 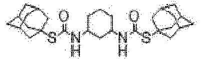 | 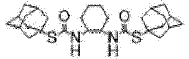 |
| **454** | **455** | **456** |
| 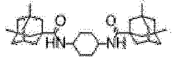 | 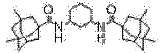 | 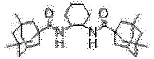 |
| **457** | **458** | **459** |
| 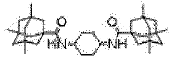 | 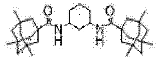 | 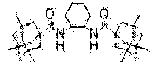 |
| **460** | **461** | **462** |
| 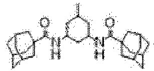 | 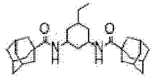 | 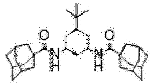 |
| **463** | **464** | **465** |
| 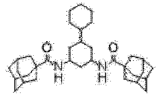 | 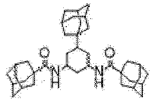 | 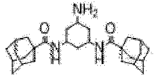 |
| **466** | **467** | **468** |
| 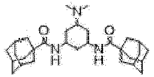 | 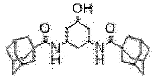 | 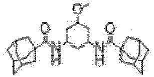 |
| **469** | **470** | **471** |
| 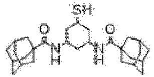 | 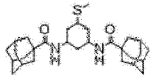 | 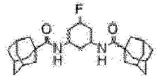 |
| **472** | **473** | **474** |
| 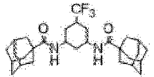 | 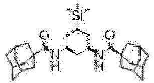 | 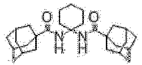 |
| **475** | **476** | **477** |
| 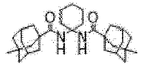 | 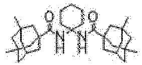 | 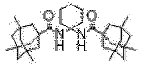 |
| **478** | **479** | **480** |
| 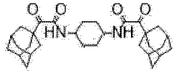 | 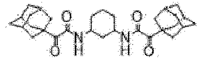 | 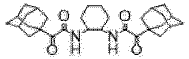 |
| **481** | **482** | **483** |
| 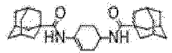 | 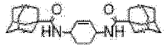 | 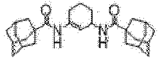 |
| **484** | **485** | **486** |
| 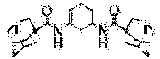 | 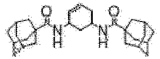 | 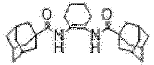 |
| **487** | **488** | **489** |
| 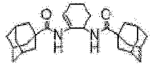 | 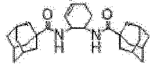 | 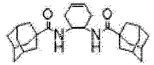 |
| **490** | **491** | **492** |
| 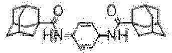 | 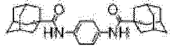 | 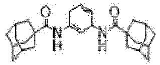 |
| **493** | **494** | **495** |
| 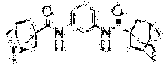 | 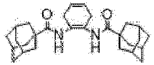 | 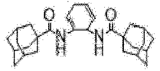 |
| **496** | **497** | **498** |
| 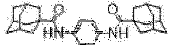 | 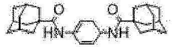 | 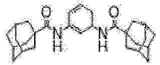 |
| **499** | **500** | **501** |
| 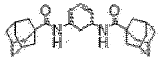 | 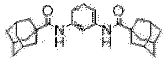 | 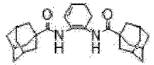 |
| **502** | **503** | **504** |
| 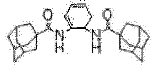 | 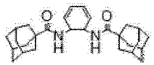 | 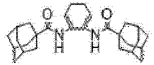 |
| **505** | **506** | **507** |
| 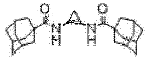 | 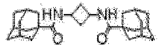 | 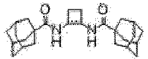 |
| **508** | **509** | **510** |
| 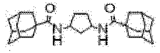 | 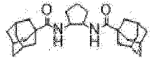 | 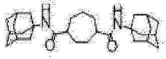 |
| **511** | **512** | **513** |
| 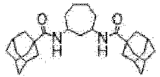 | 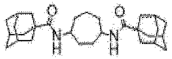 | 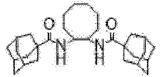 |
| **514** | **515** | **516** |
| 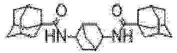 | 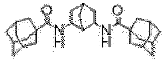 | 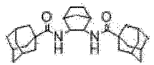 |
| **517** | **518** | **519** |
| 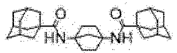 | 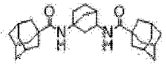 | 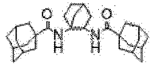 |
| **520** | **521** | **522** |
| 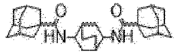 | 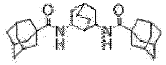 | 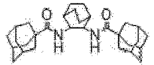 |
| **523** | **524** | **525** |
| 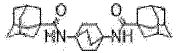 | 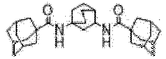 | 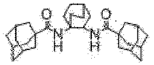 |
| **526** | **527** | **528** |
| 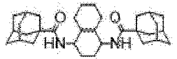 | 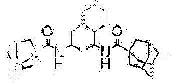 | 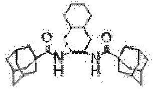 |
| **529** | **530** | **531** |
| 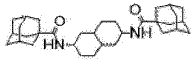 | 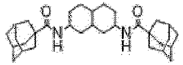 | 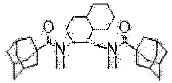 |
| **532** | **533** | **534** |
| 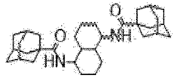 | 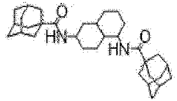 | 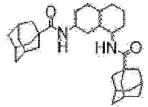 |
| **535** | **536** | **537** |
| 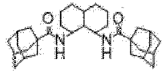 | 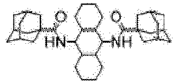 | 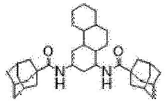 |
| **538** | **539** | **540** |
| 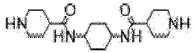 | 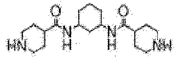 | 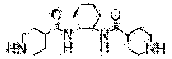 |
| **541** | **542** | **543** |
| 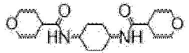 | 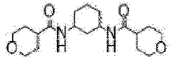 | 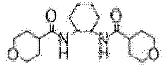 |
| **544** | **545** | **546** |
| 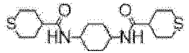 | 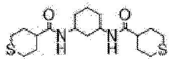 | 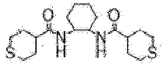 |
| **547** | **548** | **549** |
| 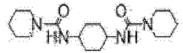 | 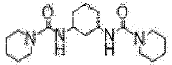 | 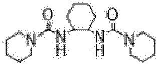 |
| **550** | **551** | **552** |
| 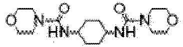 | 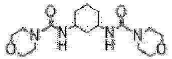 | 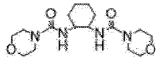 |
| **553** | **554** | **555** |
| 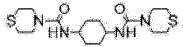 | 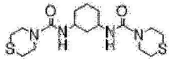 | 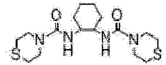 |
| **556** | **557** | **558** |
| 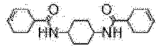 | 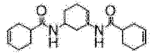 | 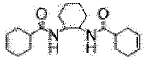 |
| **559** | **560** | **561** |
| 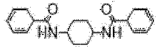 | 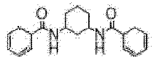 | 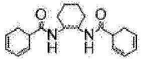 |
| **562** | **563** | **564** |
| 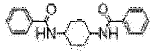 | 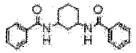 | 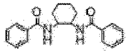 |
| **565** | **566** | **567** |
| 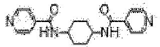 | 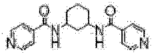 | 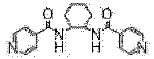 |
| **568** | **569** | **570** |
| 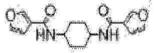 | 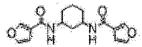 | 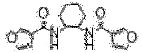 |
| **571** | **572** | **573** |
| 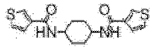 | 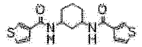 | 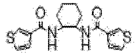 |
| **574** | **575** | **576** |
| 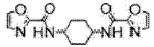 | 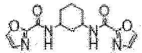 | 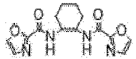 |
| **577** | **578** | **579** |
| 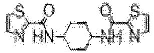 | 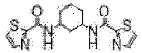 | 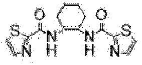 |
| **580** | **581** | **582** |
| 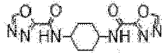 | 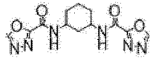 | 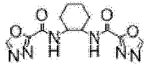 |
| **583** | **594** | **585** |
| 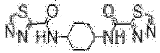 | 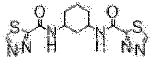 | 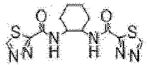 |
| **586** | **587** | **588** |
| 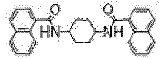 | 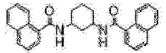 | 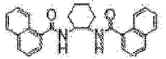 |
| **589** | **590** | **591** |
| 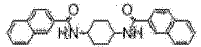 | 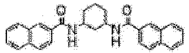 | 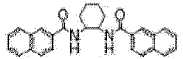 |
| **592** | **593** | **594** |
| 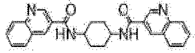 | 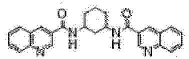 | 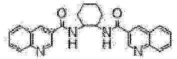 |
| **595** | **596** | **597** |
| 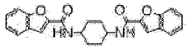 | 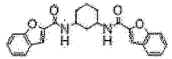 | 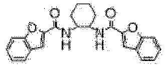 |
| **598** | **599** | **600** |
| 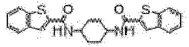 | 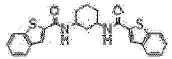 | 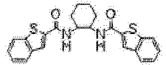 |
| **601** | **602** | **603** |
| 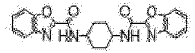 | 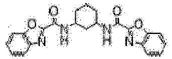 | 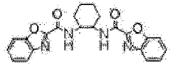 |
| **604** | **605** | **606** |
| 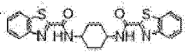 | 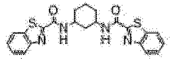 | 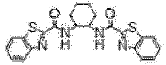 |
| **607** | **608** | **609** |
| 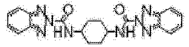 | 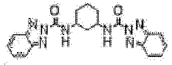 | 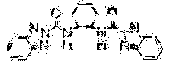 |
| **610** | **611** | **612** |
| 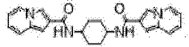 | 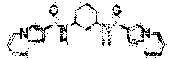 | 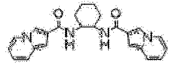 |
| **613** | **614** | **615** |
| 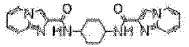 | 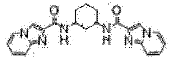 | 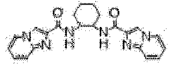 |
| **616** | **617** | **618** |
| 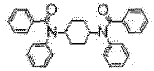 | 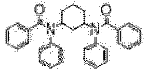 | 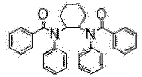 |
| **619** | **620** | **621** |
| 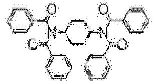 | 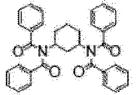 | 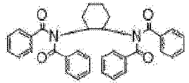 |
| **622** | **623** | **624** |
| 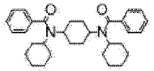 | 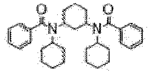 | 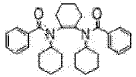 |
| **625** | **626** | **627** |
| 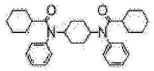 | 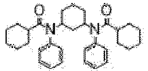 | 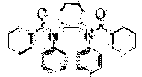 |
| **628** | **629** | **630** |
| 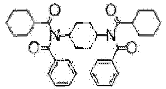 | 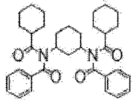 | 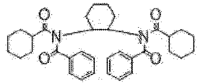 |
| **631** | **632** | **633** |
| 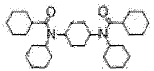 | 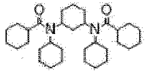 | 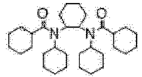 |
| **634** | **635** | **636** |
| 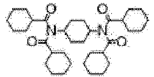 | 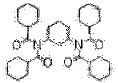 | 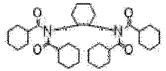 |
| **637** | **638** | **639** |
| 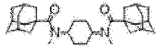 | 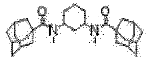 | 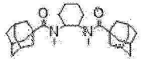 |
| **640** | **641** | **642** |
| 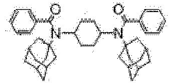 | 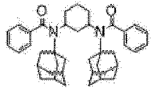 | 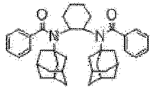 |
| **643** | **644** | **645** |
| 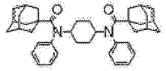 | 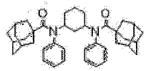 | 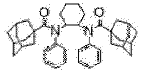 |
| **646** | **647** | **648** |
| 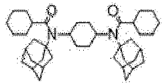 | 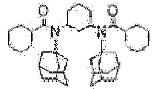 | 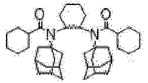 |
| **649** | **650** | **651** |
| 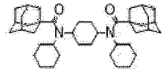 | 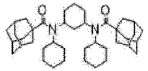 | 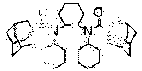 |
| **652** | **653** | **654** |
| 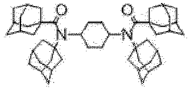 | 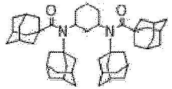 | 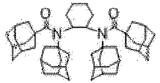 |
| **655** | **656** | **657** |
| 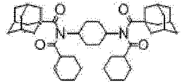 | 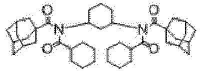 | 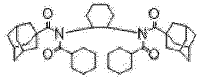 |
| **658** | **659** | **660** |
| 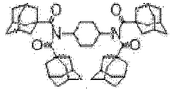 | 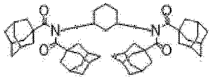 | 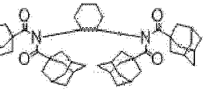 |
| **661** | **662** | **663** |
| 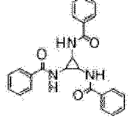 | 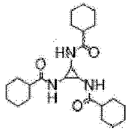 | 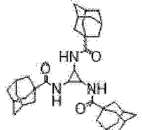 |
| **664** | **665** | **666** |
| 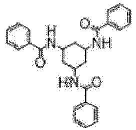 | 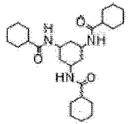 | 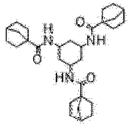 |
| **667** | **668** | **669** |
| 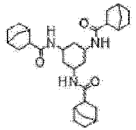 | 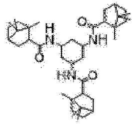 | 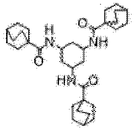 |
| **670** | **671** | **672** |
| 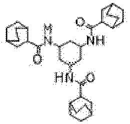 | 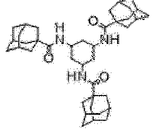 | 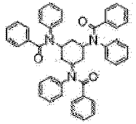 |
| **673** | **674** | **675** |
| 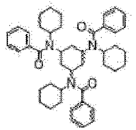 | 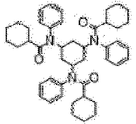 | 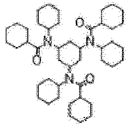 |
| **676** | **677** | **678** |
| 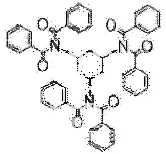 | 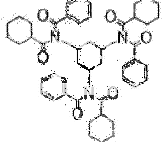 | 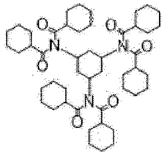 |
| **679** | **680** | **681** |
| 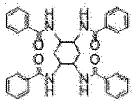 | 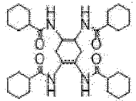 | 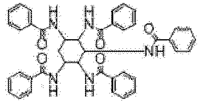 |
| **682** | **683** | **684** |
| 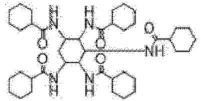 | 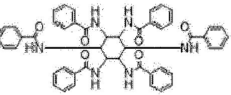 | 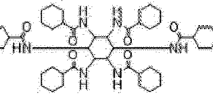 |
| **685** | **686** | **687** |
| 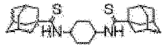 | 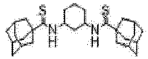 | 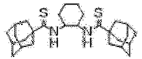 |
| **688** | **689** | **690** |
| 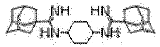 | 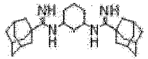 | 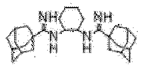 |
| **691** | **692** | **693** |
| 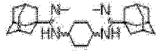 | 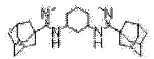 | 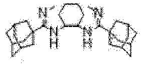 |
| **694** | **695** | **696** |
| 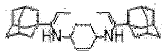 | 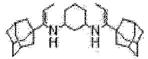 | 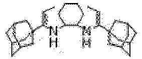 |
| **697** | **698** | **699** |
| 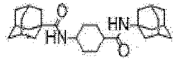 | 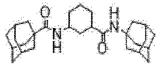 | 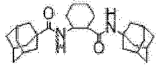 |
| **700** | **701** | **702** |
| 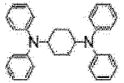 | 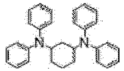 | 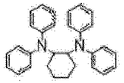 |
| **703** | **704** | **705** |
| 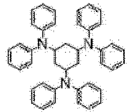 | 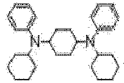 | 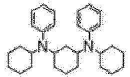 |
| **706** | **707** | **708** |
| 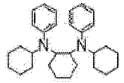 | 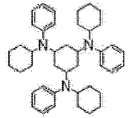 | 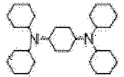 |
| **709** | **710** | **711** |
| 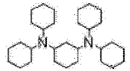 | 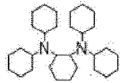 | 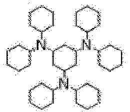 |
| **712** | **713** | **714** |
| 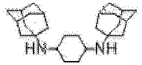 | 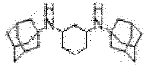 | 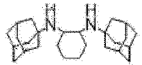 |
| **715** | **716** | **717** |
| 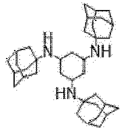 | 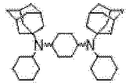 | 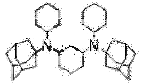 |
| **718** | **719** | **720** |
| 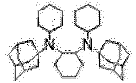 | 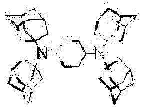 | 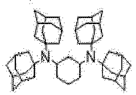 |
| **721** | **722** | **723** |
| 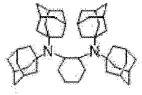 | 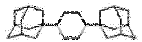 | 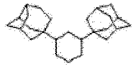 |
| **724** | **725** | **726** |
| 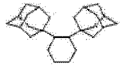 | 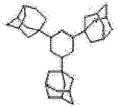 | 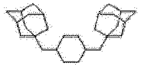 |
| **727** | **728** | **729** |
| 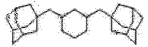 | 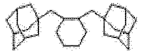 | 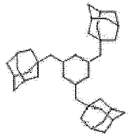 |
| **730** | **731** | **732** |
| 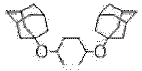 | 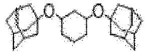 | 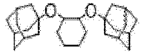 |
| **733** | **734** | **735** |
| 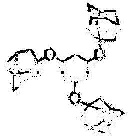 | 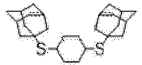 | 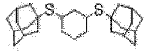 |
| **736** | **737** | **738** |
| 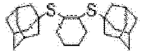 | 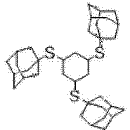 | 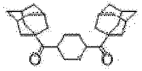 |
| **739** | **740** | **741** |
| 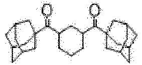 | 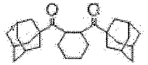 | 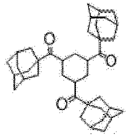 |
| **742** | **743** | **744** |

14. The compound for a capping layer of claim 1, wherein the compound has a refractive index of 1.55 or less at a wavelength of 450 nm.

15. An organic light-emitting device comprising a capping layer comprising the compound for a capping layer of claim 1.

16. The organic light-emitting device of claim 15, wherein the organic light-emitting device comprises:
a first electrode;
a second electrode; and
one or more organic layers interposed between the first electrode and the second electrode,
wherein the capping layer is disposed on an outer surface of either one of the first and electrodes.

17. The organic light-emitting device of claim 15, wherein the capping layer has a thickness of 100 Å to 3000 Å.

18. The organic light-emitting device of claim 15, wherein the capping layer has a refractive index of 1.55 or less at a wavelength of 450 nm.

19. The organic light-emitting device of claim 16, wherein the capping layer comprises a first capping layer comprising the compound of claim 1 and a second capping layer having a refractive index higher than that of the first capping layer.

20. The organic light-emitting device of claim 19, wherein the second capping layer is interposed between the first capping layer and the first electrode or between the first capping layer and said the electrode.

21. The organic light-emitting device of claim 19, wherein the second capping layer is in contact with the first capping layer and the first electrode or in contact with the first capping layer and the second electrode.

22. The organic light-emitting device of claim 19, wherein a total thickness of the first capping layer and the second capping layer is in a range of 100 Å to 3000 Å.

23. The organic light-emitting device of claim 19, wherein the first capping layer has a refractive index of 1.55 or less at a wavelength of 450 nm, the second capping layer has a refractive index of 2.10 or more at a wavelength of 450 nm, and a refractive index difference between the first capping layer and the second capping layer at a wavelength of 450 nm is in a range of 0.2 to 1.2.
